# EUROPÄISCHE PATENTSCHRIFT

(11) **EP 1 945 600 B1**
(45) Veröffentlichungstag und Bekanntmachung des Hinweises auf die Patenterteilung: **15.04.2009**
(21) Anmeldenummer: 06807555.5
(22) Anmeldetag: 26.10.2006
(51) Int. Cl.: C07C 45/33, C07C 45/35, C07C 51/215, C07C 51/25

(54) **VERFAHREN ZUM STABILEN BETREIBEN EINES KONTINUIERLICH AUSGEÜBTEN HERSTELLPROZESSES ZUR ERZEUGUNG VON ACROLEIN, ODER ACRYLSÄURE ODER DEREN GEMISCH AUS PROPAN**
METHOD FOR CARRYING OUT IN A STABLE MANNER A PROCESS FOR CONTINUOUSLY PRODUCING ACROLEIN OR ACRYLIC ACID OR THE MIXTURE THEREOF FROM PROPANE
PROCEDE PERMETTANT DE METTRE EN OEUVRE DE MANIERE STABLE UN PROCESSUS DE PRODUCTION EN CONTINU D'ACROLEINE OU D'ACIDE ACRYLIQUE OU D'UN MELANGE DES DEUX A PARTIR DE PROPANE

(30) Priorität: 03.11.2005 DE 102005052923; 03.11.2005 US 732658 P
(43) Veröffentlichungstag der Anmeldung: 23.07.2008
(73) Patentinhaber: BASF SE, 67056 Ludwigshafen (DE)
(72) Erfinder: DIETERLE, Martin, 67063 Ludwigshafen (DE); SCHINDLER, Götz-Peter, 67071 Ludwigshafen (DE); KLANNER, Catharina, 68163 Mannheim (DE)
(86) Internationale Anmeldenummer: PCT/EP2006/067784
(87) Internationale Veröffentlichungsnummer: WO 2007/051750

(56) Entgegenhaltungen:
- EP-A1- 0 117 146
- EP-B1- 0 799 169
- DE-A1- 10 245 585
- DE-A1-9102004 032 12

## Beschreibung

Vorliegende Erfindung betrifft ein Verfahren zum stabilen Betreiben eines kontinuierlich ausgeübten Herstellprozesses zur Erzeugung von Acrolein, oder Acrylsäure oder deren Gemisch aus Propan, bei dem man
A) in einer ersten Reaktionszone A Propan im Beisein von molekularem Sauerstoff einer heterogen katalysierten Dehydrierung unterwirft, wobei ein Propan und Propylen enthaltendes Produktgasgemisch A erhalten wird,
B) gegebenenfalls Produktgasgemisch A in eine erste Trennzone A führt, um in selbiger von im Produktgasgemisch A enthaltenen, von Propan und Propylen verschiedenen, Bestandteilen eine Teilmenge oder mehr abzutrennen und dadurch ein verbleibendes, Propan und Propylen enthaltendes, Produktgasgemisch A' zu erzeugen,
C) Produktgasgemisch A oder Produktgasgemisch A' in einer zweiten Reaktionszone B zur Beschickung wenigstens eines Oxidationsreaktors verwendet und in dem wenigstens einen Oxidationsreaktor im Produktgasgemisch A oder im Produktgasgemisch A' enthaltenes Propylen einer selektiven heterogen katalysierten partiellen Gasphasenoxidation mit molekularem Sauerstoff zu einem Acrolein, oder Acrylsäure, oder deren Gemisch als Zielprodukt, nicht umgesetztes Propan, überschüssigen molekularen Sauerstoff und gegebenenfalls nicht umgesetztes Propylen enthaltenden Produktgasgemisch B unterwirft,
D) in einer zweiten Trennzone B im Produktgasgemisch B enthaltenes Zielprodukt abtrennt und von dem dabei verbleibenden, Propan, molekularen Sauerstoff sowie gegebenenfalls nicht umgesetztes Propylen enthaltenden Restgas wenigstens eine nicht umgesetztes Propan, molekularen Sauerstoff und gegebenenfalls nicht umgesetztes Propylen enthaltende Teilmenge als molekularen Sauerstoff enthaltendes Kreisgas 1 in Kreisgasfahrweise in die Reaktionszone A rückführt,
E) dem kontinuierlich ausgeübten Herstellprozess über wenigstens eine der Zonen aus der Gruppe umfassend die Reaktionszone A, die Trennzone A, die Reaktionszone B und die Trennzone B Frischpropan mit einer Zufuhrrate zuführt, die im stabilen Betriebszustand des Herstellprozesses einen vorgegebenen stationären Wert aufweist, und
F) den Gehalt des Produktgasgemischs B an molekularem Sauerstoff kontinuierlich ermittelt und mit dem für den stabilen Betriebszustand des Herstellprozesses vorgegebenen stationären Zielgehalt an molekularem Sauerstoff im Produktgasgemisch B vergleicht.

Acrylsäure ist als ein Partialoxidationsprodukt des Propylen ein bedeutendes Monomeres, das als solches oder in Form seiner Alkylester zur Erzeugung von z. B. als Klebstoffe geeigneten oder Wasser superabsorbierenden Polymerisaten Verwendung findet (vgl. z. B. WO 02/055469 und WO 03/078378). Acrolein ist ein bedeutendes Zwischenprodukt, beispielsweise für die Herstellung von Glutardialdehyd, Methionin, Folsäure und Acrylsäure.

Im Unterschied zur exotherm verlaufenden heterogen katalysierten Oxidehydrierung von Propan, die durch anwesenden Sauerstoff erzwungen und bei der intermediär kein freier Wasserstoff gebildet wird (der dem zu dehydrierenden Propan entrissene Wasserstoff wird unmittelbar als Wasser (H₂O) entrissen) bzw. nachweisbar ist, soll dabei in dieser Schrift unter einer heterogen katalysierten Dehydrierung des Propans eine ("konventionelle") Dehydrierung verstanden werden, deren Wärmetönung im Unterschied zur Oxidehydrierung endotherm ist (als Folgeschritt kann eine exotherme Wasserstoffverbrennung in die heterogen katalysierte Dehydrierung in der ersten Reaktionszone A einbezogen sein) und bei der wenigstens intermediär freier molekularer Wasserstoff gebildet wird. Dazu bedarf es in der Regel anderer Reaktionsbedingungen und anderer Katalysatoren als zur Oxidehydrierung.

In entsprechender Weise wird in dieser Schrift unter Frischpropan Propan verstanden, das weder an einer Dehydrierung in der Reaktionszone A, noch an einer Partialoxidation von Propylen zu Acrolein und/oder Acrylsäure in der Reaktionszone B teilgenommen hat. Vorzugsweise hat es überhaupt noch an keiner chemischen Reaktion teilgenommen. In der Regel wird es in Form von Roh-Propan zugeführt, das vorzugsweise die Spezifikation gemäß der DE-A 102 46 119 bzw. der DE-A 102 45 585 erfüllt, und das normalerweise in geringen Mengen auch von Propan verschiedene Komponenten enthält. Solches Roh-Propan ist beispielsweise nach in der DE-A 10 2005 022 798 beschriebenen Verfahren erhältlich. Normalerweise enthält Roh-Propan zu wenigstens ≥ 90 Gew.-% und vorzugsweise zu wenigstens 95 Gew.-% Propan.

Als Inertgas soll in dieser Schrift ganz generell ein Reaktionsgasbestandteil verstanden werden, der sich unter den Bedingungen der entsprechenden Reaktion im wesentlichen als inert verhält und -jeder inerte Reaktionsgasbestandteil für sich betrachtet - zu mehr als 95 mol-%, vorzugsweise zu mehr als 98 mol-% und besonders bevorzugt zu mehr als 99 mol-% chemisch unverändert erhalten bleibt. Beispiele für solche Inertgase in den Reaktionszonen A und B sind N₂, Edelgase, CO₂ oder auch Wasserdampf.

Unter der Belastung eines einen Reaktionsschritt katalysierenden Katalysatorbetts mit Reaktionsgasgemisch wird in dieser Schrift die Menge an Reaktionsgasgemisch in Normlitern (= Nl; das Volumen in Litern, das die entsprechende Reaktionsgasgemischmenge bei Normalbedingungen (0°C, 1 bar) einnehmen würde) verstanden, die pro Stunde durch einen Liter Katalysatorbett (z. B. Katalysatorfestbett) geführt wird. Die Belastung kann aber auch nur auf einen Bestandteil des Reaktionsgasgemischs bezogen sein. Dann ist es die Menge dieses Bestandteils in NI/I·h, die pro Stunde durch einen Liter des Katalysatorbetts geführt wird (reine Inertmatieralschüttungen werden nicht zum Katalysatorfestbett gerechnet). Die Belastung kann auch nur auf die in einem Katalysatorbett, das den eigentlichen Katalysator mit Inertmaterial verdünnt enthalten kann, enthaltene Menge an Katalysator bezogen sein (dies wird dann explizit vermerkt).

Verfahren zur Herstellung von Acrolein und/oder Acrylsäure, bei denen man das Propylen durch partielle heterogen katalysierte Dehydrierung aus Propan erzeugt und nachfolgend im Beisein von nicht umgesetztem (inertem) Propan als Bestandteil eines Partialoxidationsgemischs einer heterogen katalysierten partiellen Gasphasenoxidation mit molekularem Sauerstoff zu Acrolein und/oder Acrylsäure enthaltenden Produktgasgemischen unterwirft, sind bekannt (vgl. z. B. DE-A 10 2005 022 798. DE-A 102 46 119, DE-A 102 45 585, DE-A 10 2005 049 699, DE-A 10 2004 032 129, DE-A 10 2005 013 039, DE-A 10 2005 010 111, DE-A 10 2005 009 891, DE-A 102 11 275, EP-A 117 146, US-A 3,161,670, DE-A 33 13 573, WO 01/96270, DE-A 103 16 039, DE-A 10 2005 009 885 und der in diesen Schriften zitierte Stand der Technik).

Es ist aus dem vorgenannten Stand der Technik auch bekannt, dass es mit Blick auf die Lebensdauer der verwendeten Katalysatoren günstig ist, wenn das Produktgasgemisch B in der Partialoxidation noch nicht umgesetzten molekularen Restsauerstoff enthält.

Es ist aus besagtem Stand der Technik aber auch bekannt, dass mit der Ziefproduktabtrennung in der Trennzone B in der Regel keine oder zumindest keine vollständige Trennung von im Produktgasgemisch B enthaltenem molekularen Sauerstoff und Propan einhergeht. Daraus resultierend enthält das Kreisgas 1 ebenfalls molekularen Sauerstoff, der in der Reaktionszone A normalerweise eine partielle Vollverbrennung von Propan und Propylen bedingt, was in der Reaktionszone A sowohl die Selektivität der Propylenbildung als auch die Wärmebilanz beeinflusst.

Ein stabiler, auf langfristige Produktion angelegter Betrieb eines wie eingangs beschriebenen, kontinuierlich ausgeübten Herstellprozesses zur Erzeugung von Acrolein, oder Acrylsäure oder deren Gemisch aus Propan setzt daher einen stabilen Sauerstoffgehalt des Produktgasgemisches B voraus. In Abhängigkeit von den in den Reaktionszonen A und B verwendeten Katalysatoren und Reaktions- und Verfahrenbedingungen kann der vorgenannte (Ziel)Sauerstoffgehalt des Produktgasgemisches B im stationären Betriebszustand des Herstellprozesses z. B. 0,1 bis 6 Vol.-%, bevorzugt 0,5 bis 5 Vol.-%, und besonders bevorzugt 1,5 bis 4 Vol.-%, z. B. auch 3 Vol.-% betragen. Der für den stationären Betrieb des Herstellprozesses vorgegebene stationäre ,Wert" des Sauerstoffgehaltes im Produktgasgemisch B kann aber auch ein Intervall (z. B. ± 0,3 Vol.-%, oder ± 0,2 Vol.-%, oder ± 0,1 Vol.-%) um einen Mittelwert sein (z. B. 3 ± 0,1 Vol.-%; in diesem Fall würde nur dann geregelt, wenn der aktuelle Zeitwert das Intervall um den Mittelwert herum verlässt).

Eingehende Untersuchungen des stationären Betriebes eines wie beschrieben ausgeübten Herstellprozesses haben ergeben, dass der Sauerstoffgehalt des Produktgasgemischs B in der Praxis vielfach temporär vom vorgegebenen (angestrebten) stationären Wert (dem Zielgehalt) als Führungsgröße (Regelgröße) abweicht, ohne das eine Abweichung der Zufuhrrate an molekularem Sauerstoff für die Reaktionszone B von ihrem Idealwert (stationären Wert) vorliegt.

Derartige Abweichungen sind in typischer Weise dadurch gekennzeichnet, dass sie vergleichsweise beschränkt sind und in der Regel durch geringfügige Abweichungen verschiedener Elemente des Gesamtverfahrens von der Ideallinie bedingt werden. Üblicherweise bewegen sie sich in einem Abweichungsumfang von teilweise ± 20 %, vielfach ± 10 %, häufig ± 5 %, oder ± 3 %, jeweils bezogen auf den stationären Zielgehalt (bzw. den Mittelwert des stationären Zielgehaltintervalls). Bei den vorgenannten Umfang verlassenden Abweichungen handelt es sich in der Regel um Störungen der Sauerstoffzufuhr zum Herstellprozess (oder um andere größere Störungen), die dann durch unmittelbare Änderung derselben beseitigt werden müssen.

Für Abweichungen im genannten Umfang bedarf es eines geeigneten anderen Stellgliedes, das dem Abweichen des Sauerstoffgehaltes im Produktgasgemisch B vom vorgegebenen stationären Wert entgegenwirkt und den Herstellungsprozess in den stationären Betriebszustand zurückführt (d.h., ein weitergehendes Weglaufen aus dem stationären Zustand frühzeitig unterbindet). Wesentlich ist dabei, dass die Stellgröße nicht zu träge reagiert. Eigene Untersuchungen haben z. B. ergeben, dass z. B. Veränderungen von Reaktionstemperaturen, die den Sauerstoffverbrauch im Hestellprozess beeinflussen, im Regelfall als Stellgrößen relativ träge reagieren, da sowohl die Zufuhr als auch der Entzug von Wärme in den bzw. aus dem Herstellprozess infolge der vergleichsweise endlichen Wärmeübergangszahlen relativ langsam erfolgen.

Die Aufgabe der vorliegenden Erfindung bestand daher darin, ein Verfahren zum stabilen Betreiben eines wie eingangs beschriebenen, kontinuierlich ausgeübten Herstellprozesses zur Erzeugung von Acrolein, oder Acrylsäure oder deren Gemisch aus Propan mit einer geeigneten Stellgröße für den Gehalt an molekularem Sauerstoff im Produktgasgemisch B als Regelgröße zur Verfügung zu stellen.

Demgemäß wurde ein Verfahren zum stabilen Betreiben eines kontinuierlich ausgeübten Herstellprozesses zur Erzeugung von Acrolein, oder Acrylsäure oder deren Gemisch aus Propan, bei dem man
A) in einer ersten Reaktionszone A Propan im Beisein von molekularem Sauerstoff einer heterogen katalysierten Dehydrierung unterwirft, wobei ein Propan und Propylen enthaltendes Produktgasgemisch A erhalten wird,
B) gegebenenfalls Produktgasgemisch A in eine erste Trennzone A führt, um in selbiger von im Produktgasgemisch A enthaltenen, von Propan und Propylen verschiedenen, Bestandteilen eine Teilmenge oder mehr abzutrennen und dadurch ein verbleibendes, Propan und Propylen enthaltendes, Produktgasgemisch A' zu erzeugen,
C) Produktgasgemisch A oder Produktgasgemisch A' in einer zweiten Reaktionszone B zur Beschickung wenigstens eines Oxidationsreaktors verwendet und in dem wenigstens einen Oxidationsreaktor im Produktgasgemisch A oder im Produktgasgemisch A' enthaltenes Propylen einer selektiven heterogen katalysierten partiellen Gasphasenoxidation mit molekularem Sauerstoff zu einem Acrolein, oder Acrylsäure, oder deren Gemisch als Zielprodukt, nicht umgesetztes Propan, überschüssigen molekularen Sauerstoff und gegebenenfalls nicht umgesetztes Propylen enthaltenden Produktgasgemisch B unterwirft,
D) in einer zweiten Trennzone B im Produktgasgemisch B enthaltenes Zielprodukt, abtrennt und von dem dabei verbleibenden, Propan, molekularen Sauerstoff sowie gegebenenfalls nicht umgesetztes Propylen enthaltenden Restgas wenigstens eine nicht umgesetztes Propan, molekularen Sauerstoff und gegebenenfalls nicht umgesetztes Propylen enthaltende Teilmenge als molekularen Sauerstoff enthaltendes Kreisgas 1 in Kreisgasfahrweise in die Reaktionszone A rückführt,
E) dem kontinuierlich ausgeübten Herstellprozess über wenigstens eine der Zonen aus der Gruppe umfassend die Reaktionszone A, die Trennzone A, die Reaktionszone B und die Trennzone B Frischpropan mit einer Zufuhrrate zuführt, die im stabilen Betriebszustand des Herstellprozesses einen vorgegebenen stationären Wert aufweist, und
F) den Gehalt des Produktgasgemischs B an molekularem Sauerstoff kontinuierlich ermittelt und mit dem für den stabilen Betriebszustand des Herstellprozesses vorgegebenen stationären Zielgehalt an molekularem Sauerstoff im Produktgasgemisch B vergleicht, gefunden,
   das dadurch gekennzeichnet ist, dass man
   - für den Fall, dass der zu einem Zeitpunkt ermittelte Gehalt an molekularem Sauerstoff im Produktgasgemisch B größer als der stationäre Zielgehalt ist, im Anschluss an diesen Zeitpunkt dem Herstellprozess Frischpropan mit einer Zufuhrrate (die Zufuhrrate des Frischpropan ist die erfindungsgemäß ausgewählte Stellgröße) zuführt, die größer als ihr stationärer Wert ist, und
   - für den Fall, dass der zu einem Zeitpunkt ermittelte Gehalt an molekularem Sauerstoff im Produktgasgemisch B kleiner als der stationäre Zielgehalt ist, im Anschluss an diesen Zeitpunkt dem Herstellprozess Frischpropan mit einer Zufuhrrate zuführt, die kleiner als ihr stationärer Wert ist.

Abgesehen vom kennzeichnenden Teil, kann das erfindungsgemäße Verfahren wie die in den Schriften EP-A 799 169, US-A 4,788,371, US-A 6,566,573, DE-A 10 2005 022 798, DE-A 102 46 119, DE-A 102 45 585, DE-A 10 2005 049 699, DE-A 10 2004 032 129, DE-A 10 2005 013 039, DE-A 10 2005 010 111, DE-A 10 2005 009 891, DE-A 102 11 275, EP-A 117 146, US-A 3,161,670, DE-A 33 13 573, WO 01/96270, DE-A 103 16 039, DE-A 10 2005 009 885 und dem in diesen Schriften zitierten Stand der Technik beschriebenen Herstellprozesse durchgeführt werden.

Unter der Zufuhrrate an Frischpropan wird beim erfindungsgemäßen Verfahren diejenige Menge an Propan verstanden, die dem Herstellprozess pro Zeiteinheit insgesamt frisch (d. h., ohne zuvor an einer Dehydrierung in der Reaktionszone A oder an einer Partialoxidation in der Reaktionszone B teilgenommen zu haben (besonders bevorzugt hat es zuvor noch an überhaupt keiner chemischen Reaktion teilgenommen)) zugeführt wird. Die Vorteilhaftigkeit der Zufuhrrate an Frischpropan als erfindungsgemäß ausgewählter Stellgröße besteht darin, dass infolge der hohen Strömungsgeschwindigkeit der Reaktionsgase innerhalb des Herstellprozesses so eine rasche Rückführung des Herstellprozesses in den stationären Betriebszustand gewährleistet ist. Das Rückführungsprinzip basiert dabei darauf, dass mit einer erhöhten Zufuhrrate an Frischpropan unter ansonsten im wesentlichen unveränderten Herstellprozessbedingungen eine vergleichsweise erhöhte Bildungsrate an Propylen in der Reaktionszone A einhergeht. Daraus resultiert eine erhöhte Propylenzufuhrrate in die Reaktionszone B, die den im Vergleich zum stationären Betriebszustand im Produktgasgemisch B erhöhten Sauerstoffgehalt durch entsprechende Umsetzung mit molekularem Sauerstoff in der Reaktionszone B wieder zum für den Herstellprozess vorgegebenen stationären Zielgehalt hinführt. Umgekehrt geht mit einer verringerten Zufuhrrate an Frischpropan unter ansonsten im wesentlichen unveränderten Herstellprozessbedingungen eine vergleichsweise erniedrigte Bildungsrate an Propylen in der Reaktionszone A einher. Daraus resultiert eine verringerte (verglichen mit dem stationären Betriebszustand) Propylenzufuhrrate in die Reaktionszone B, die den im Vergleich zum stationären Betriebszustand im Produktgasgemisch B erniedrigten Sauerstoffgehalt durch in entsprechender Weise in geringerem Umfang erfolgende Umsetzung mit molekularem Sauerstoff in der Reaktionszone B wieder zum für den Herstellprozess vorgegebenen stationären Zielgehalt hinführt. Zusätzlich geht mit einer veränderten Propylenbildungsrate in der Reaktionszone A eine veränderte Endothermie der Propylenbildung in selbiger einher, die eine durch den veränderten O₂-Gehalt in der Reaktionszone A veränderte Verbrennungsexothermie in selbiger zu nivellieren vermag (dieser Vorteil erwächst insbesondere dann, wenn die Änderung der Zufuhrrate an Frischpropan unmittelbar in die Reaktionszone A hinein erfolgt; dies vor allem dann, wenn sie unmittelbar in das der Reaktionszone A zugeführte Reaktionsgasgemischeingangsgas hinein erfolgt). Darüber hinaus in der Reaktionszone A gegebenenfalls einhergehende Änderungen der Reaktionstemperatur in der Reaktionszone A können bei Bedarf durch entsprechende Änderung der Temperatur z. B. der der Reaktionszone A zugeführten Gase nachgeregelt werden. Die veränderte Zufuhrrate an Frischpropan bleibt beim erfindungsgemäßen Verfahren in der Regel so lange aufrechterhalten, bis die Abweichung des Gehaltes an molekularem Sauerstoff im Produktgasgemisch B vom Zielgehalt ihr Vorzeichen zu ändern beginnt usw..

Vorteilhaft ist an der Zufuhrrate an Frischpropan als erfindungsgemäß ausgewählter Stellgröße ferner, dass mit ihrer Veränderung lediglich eine vergleichsweise begrenzte Veränderung der Reaktionsgasgesamtstromrate einhergeht. Dies ist dadurch bedingt, dass mit einer Zufuhr an Frischpropan nicht in notwendiger Weise eine signifikante Zufuhr an von Propan verschiedenen Komponenten einhergeht. Letzteres wäre jedoch z. B. dann der Fall, wenn einem veränderten Restsauerstoffgehalt in der Reaktionszone B mit einer variierenden Luftzudosierung unmittelbar in die Reaktionszone A hinein begegnet würde, da Luftsauerstoff immer von der vierfachen molaren Menge an Stickstoff begleitet wird. Die Bereitstellung von reinem molekularem Sauerstoff (als alternative Stellgröße) ist jedoch vergleichsweise aufwendig.

Das Vorgenannte trifft vor allem dann zu, wenn der vorgegebene stationäre Zielgehalt des Produktgasgemischs B an molekularem Sauerstoff in Vol.-% und bezogen auf das Gesamtvolumen des Produktgasgemisches B vorgegeben wird.

Es trifft aber auch dann zu, wenn der vorgegebene stationäre Zielgehalt des Produktgasgemischs B an molekularem Sauerstoff in Gew.-% und bezogen auf das Gesamtgewicht des Produktgasgemischs B vorgegeben wird.

Die Ermittlung des Gehalts an molekularem Sauerstoff im Produktgasgemisch B kann auf unterschiedlichste Art und Weise erfolgen. Erfindungsgemäß bevorzugt erfolgt sie online. Beispielsweise kann die in der DE-A 101 17 678 veröffentlichte Methode angewendet werden, die darauf beruht, dass ein Signal gewonnen wird, das mit dem Sauerstoffgehalt des Produktgasgemischs B korreliert ist. Beispielsweise kann das Signal dadurch gewonnen werden, dass elektromagnetische Strahlung mit einer Wellenlänge λₒ(z. B. im IR-Bereich liegend), bei der molekularer Sauerstoff elektromagnetische Strahlung absorbiert (z. B. 759,5 bis 768 nm), durch (das) Produktgasgemisch B geleitet und der nichtabsorbierte Anteil der elektromagnetischen Strahlung gemessen wird. Beispielsweise kann der Sauerstoffgehalt von Produktgasgemisch B mittels eines Laserstrahls gemessen werden, dessen Wellenlänge auf eine der Rotations-Feinstruktur-Banden von molekularem Sauerstoff eingestellt ist. Zum Eichen der Messung des Sauerstoffgehalts kann der Laserstrahl eine Kalibrierzelle durchstrahlen, die ein Gas (z. B. im stationären Zustand befindliches Produktgasgemisch B) mit definiertem Sauerstoffgehalt enthält oder durch die ein Gas mit definiertem Sauerstoffgehalt geleitet wird. Beispielsweise kann der vorgenannte Laser ein Diodenlaser sein, dessen Wellenlänge auf eine der Rotations-Feinstruktur-Banden von molekularem Sauerstoff im Bereich von 759,5 nm bis 768 nm einstellbar ist. Der Modulationsbereich kann dabei ± 0,05 nm betragen. Prinzipiell kann die Messung unmittelbar, z. B. durch ein entsprechendes Fenster hindurch, im Produktgasgemisch B selbst durchgeführt werden. Es kann über einen Bypass aber auch ein kleiner Mengenstrom des Produktgasgemischs B (mit gleicher Zusammensetzung) zum besagten Messzweck abgezweigt werden, wie es z. B. die DE-A 101 17 678 beschreibt und empfiehlt.

Grundsätzlich kann für den beschriebenen Zweck z. B. ein Gasanalysengerät vom Typ Ultramat^{®} 23 der Firma Siemens eingesetzt werden (arbeitet im IR-Bereich). In Betracht kommen z. B. die Geräte 7MB2335, 7MB2337 und 7MB2338 (vgl. die Broschüre ,Siemens, Ultramat 23, Betriebsanleitung, Bestell-Nr.: C79000-B5200-C216-02, Version 01/2005").

Für das erfindungsgemäße Verfahren geeignet sind aber auch Messverfahren und -geräte, die auf der vergleichsweise großen paramagnetischen Suszeptibilität des molekularen Sauerstoffs basieren, wie z. B. Sauerstoff-Analysatoren der Serie PMA^{®} der Fa. M&C Products Analysentechnik GmbH in D-40885 Ratingen. Hierbei handelt es sich um Analysengeräte, die aufgrund ihrer sehr schnellen Ansprechzeit, ihres geringen Totvolumens, ihrer geringen Querempfindlichkeit gegenüber anderen Gasbestandteilen sowie der direkten Durchströmbarkeit der Messzelle besonders vorteilhaft sind (z.B. die Version PMA 30). Die Messverfahren auf der Grundlage des vorgenannten Prinzips gehören zu den genauesten quantitativen Bestimmungsverfahren für Sauerstoff im Bereich von 0 bis 100 Vol.-%. An Spannbändern ist bei den vorgenannten Geräten eine diamagnetische Hantel mit im Drehpunkt befindlichem Spiegel befestigt und in einem inhomogenen Magnetfeld montiert. Der Sauerstoff strebt infolge seines Paramagnetismus in das inhomogene Magnetfeld der Messzelle. Die O₂-Moleküle üben dabei auf die Hantel ein Drehmoment aus und lenken sie aus. Durch optische Abtastung wird elektronisch ein Strom erzeugt, der durch eine Drahtschleife fließt, die um die Hantel gelegt ist und diese in die neutrale Lage zurück dreht. Der Kompensationsstrom ist proportional zum Sauerstoffgehalt des Messgases, wodurch die O₂-Anzeige absolut linear ist.

Als O₂-Bestimmungsmethode kommen aber für das erfindungsgemäße Verfahren prinzipiell auch gaschromatische Methoden oder elektrochemische Verfahren in Betracht. Bei letzteren wird der molekulare Sauerstoff z. B. durch geeignete Energiezufuhr ionisiert, und anschließend die elektrische Leitfähigkeit des Gases bestimmt.

Wie bereits erwähnt, kann der stationäre Zielgehalt an molekularem Sauerstoff im Produktgasgemisch B beim erfindungsgemäßen Verfahren beispielsweise auf 0,1 bis 6 Vol.-%, bevorzugt auf 0,5 bis 5 Vol.-%, besonders bevorzugt auf 1,5 bis 4 Vol.-% und z.B. auch auf 3 Vol.-% festgelegt sein. Vorstehende Festlegung kann sich aber auch auf 0,1 bis 6 Gew.-%, oder 0,5 bis 5 Gew.-%, oder 1,5 bis 4 Gew.-%, oder z.B. auch auf 3 Gew.-% erstrecken.

Weicht der tatsächliche Sauerstoffgehalt des Produktgasgemischs B vom vorgegebenen stationären Zielgehalt ab, wird erfindungsgemäß die Zufuhrrate an Frischpropan entsprechend angepasst. Die Anpassung erfolgt dabei erfindungsgemäß zweckmäßig so, dass die daraus resultierende veränderte Propylenbildungsrate in der Reaktionszone A so bemessen ist, dass in der Reaktionszone B, bezogen auf die Partialoxidation im stationären Betrieb, die Sauerstoffgehaltabweichung durch eine reaktionsstöchiometrisch äquivalente Propylengehaltabweichung im wesentlichen gerade jeweils kompensiert wird.

Grundsätzlich kann beim erfindungsgemäßen Verfahren die Zufuhr des für den Herstellprozess benötigten Frischpropan über jede der Zonen aus der Gruppe umfassend die Reaktionszone A, die Trennzone A, die Reaktionszone B und die Trennzone B erfolgen. Dies vor allem dann, wenn das Kreisgas 1 die Gesamtmenge des in der Trennzone B verbleibenden, Propan und molekularen Sauerstoff sowie gegebenenfalls Propylen enthaltenden Restgases umfasst.

Mit Vorteil erfolgt die Frischpropanzufuhr für den Herstellprozess des erfindungsgemäßen Verfahrens ausschließlich in die Reaktionszone A hinein (dies bedingt in der Regel die höchsten Zielproduktselektivitäten bezogen auf zugeführtes Propan). Sie kann aber auch in jede andere der verschiedenen Zonen und auch über mehr als eine Zone gleichzeitig zugeführt werden (insbesondere eine Zufuhr an Frischpropan unmittelbar vor der Reaktionszone B kann z. B. dann zweckmäßig sein, wenn dadurch eine Stabilisierung des Explosionsverhaltens des Reaktionsgasgemischs in der Partialoxidationszone (Reaktionszone B) bewirkt wird). In entsprechender Weise kann beim erfindungsgemäßen Verfahren die erfindungsgemäße Veränderung der Zufuhrrate an Frischpropan in jede der Zonen hinein erfolgen.

Erfolgt die Frischpropanzufuhr für den Herstellprozess des erfindungsgemäßen Verfahrens ausschließlich in die Reaktionszone A hinein, wird die erfindungsgemäße Veränderung der Zufuhrrate an Frischpropan erfindungsgemäß zweckmäßig auch ausschließlich in die Reaktionszone A hinein, d.h., an derselben Zufuhrstelle vorgenommen. Im Übrigen ist es (wie bereits erwähnt) generell vorteilhaft, die Änderung der Zufuhrrate an Frischpropan in die Reaktionszone A hinein vorzunehmen.

Darüber hinaus kann der dem erfindungsgemäßen Verfahren zugrunde liegende Herstellprozess, wie bereits erwähnt, wie in den Schriften des gewürdigten Standes der Technik beschrieben ausgeführt werden.

In den Schriften DE-A 10 2004 032 129 und DE-A 10 2005 013 039 wurde diesbezüglich vorgeschlagen, dass der Reaktionszone A als Reaktionsgasgemischeingangsgas ein Gemisch aus Wasserdampf, Frischpropan und Kreisgas 1 zum Zweck der partiellen heterogen katalysierten Dehydrierung des im Reaktionsgasgemischeingangsgas enthaltenen Propan zu Propylen zugeführt wird. Dabei sollte die heterogen katalysierte Propandehydrierung zweckmäßig als Hordenreaktor verwirklicht sein, in welchem die Katalysatorbetten in günstiger Weise radial oder axial hintereinander angeordnet sind. Zweckmäßigerweise wird in einem solchen Hordenreaktor der Katalysatorfestbetttyp angewendet. In vorteilhafter Weise liegt die Anzahl der Katalysatorbetthorden in einem solchen Hordenreaktor bei drei. Dabei empfiehlt der Stand der Technik, die heterogen katalysierte partielle Propandehydrierung autotherm durchzuführen. Dazu wird dem Reaktionsgasgemisch hinter dem ersten durchlaufenen Katalysatorbett und zwischen den auf das in Strömungsrichtung erste Katalysator(fest)bett nachfolgenden Katalysator(fest)betten in begrenztem Umfang molekularer Sauerstoff (z. B. in Form von Luft) zugesetzt. So kann, in der Regel durch die Dehydrierkatalysatoren selbst katalysiert, eine begrenzte Verbrennung von im Verlauf der heterogen katalysierten Propandehydrierung gebildetem Wasserstoff (sowie gegebenenfalls in höchstens geringem Umfang von Propan und/oder Propylen) bewirkt werden, deren exotherme Wärmetönung die Dehydriertemperatur im wesentlichen erhält (adiabate Reaktorgestaltung).

In den Vergleichsbeispielen 1, 3 und 4 der DE-A 10 2004 032 129 sowie im Ausführungsbeispiel der DE-A 10 2005 010 111 wird eine solche wie vorstehend beschriebene heterogen katalysierte partielle Propandehydrierung im Hordenreaktor (3 Katalysatorbetthorden) durch drei hintereinandergeschaltete Dehydrierreaktoren simuliert. Anstelle einer solchen Hintereinanderschaltung von Rohrreaktoren kann in diesen Vergleichsbeispielen bzw. in diesem Ausführungsbeispiel aber auch ein Hordenreaktor gemäß den Ausführungen der DE-A 10 2005 049 699 verwendet werden.

Als Katalysator ist dabei jeweils der Katalysator des entsprechenden Vergleichsbeispiels bzw. des Ausführungsbeispiels zu verwenden. Das gleiche gilt für die Reaktionstemperaturen und die Zusammensetzung des Reaktionsgasgemischeingangsgases.

Zweckmäßig wird die partielle heterogen katalysierte Dehydrierung des Propans dabei im wesentlichen auf die drei Katalysatorhorden verteilt so betrieben, dass der Umsatz des in den Reaktor geführten Propans, bezogen auf einmaligen Reaktordurchgang, ca. 20 mol.-% beträgt. Die erzielte Selektivität der Propylenbildung liegt dabei regelmäßig bei 90 mol.-%. Der maximale Umsatzbeitrag einer einzelnen Horde wandert mit zunehmender Betriebsdauer in Strömungsrichtung von vorne nach hinten. In der Regel wird die Katylsatorbeschickung regeneriert, bevor die in Strömungsrichtung dritte Horde den maximalen Umsatzbeitrag erbringt. Mit Vorteil erfolgt die Regenerierung dann, wenn die Verkokung aller Horden ein identisches Ausmaß erreicht hat.

Günstig ist es für die vorbeschriebene heterogen katalysierte partielle Dehydrierung des Propan ganz generell, wenn die Belastung der Katalysatorgesamtmenge (Summe über alle Betten) mit der Gesamtmenge aus Propan und Propylen ≥ 500 NI/I·h und ≤ 20000 NIII-H beträgt (typische Werte sind 1500 NIII-H bis 2500 NI/i-h). Die maximale Reaktionstemperatur innerhalb eines individuellen Katalysatorfestbetts wird dabei mit Vorteil bei 500°C bis 600°C (bzw. bis 650°C) gehalten. Mit besonderem Vorteil besteht das Reaktionsgasgemischeingangsgas bei vorbeschriebener heterogen katalysierter partieller Propandehydrierstufe im Hordenreaktor lediglich aus Frischpropan und dem aus der Partialoxidation in die Dehydrierung rückgeführten Kreisgas 1, das von der Partialoxidation herrührend eine ausreichende Menge an Wasserdampf enthält, um eine befriedigende Standzeit der Dehydrierkatalysatorbetten zu bedingen. D.h., die Vergleichsbeispiele und das Beispiel sind in den beschriebenen Hordenreaktoren auch dann so durchführbar, wenn auf den Zusatz des Extrawasserdampf in der Dehydrierung verzichtet wird. Im übrigen gelten die in den Schriften DE-A 10 2005 009 885, DE-A 10 2005 010 111, DE-A 10 2005 049 699, DE-A 10 2005 009 891, DE-A 10 2005 013 039 und DE-A 10 2004 032 129 zu solchen Verfahrensweisen gemachten Aussagen.

Nachteilig am vorstehend beschriebenen Verfahren ist, dass nahezu alle Katalysatoren, die die Dehydrierung des Propan katalysieren, auch die Verbrennung (vollständige Oxidation von Propan und Propylen zu Kohlenoxiden und Wasserdampf) von Propan und Propylen mit molekularem Sauerstoff katalysieren und, wie bereits ausgeführt, molekularer Sauerstoff im in die heterogen katalysierte partielle Dehydrierung des Propan rückgeführten Kreisgas 1 aus der Partialoxidation enthalten ist.

Es wurde deshalb auch schon vorgeschlagen (siehe DE-A 102 11 275), im Rahmen einer erfindungsgemäßen Herstellung von Acrolein und/oder Acrylsäure aus Propan die heterogen katalysierte partielle Propandehydrierung so auszuführen, dass man das der Dehydrierzone entnommene Produktgas in zwei Teilmengen identischer Zusammensetzung aufteilt, um nur eine der beiden Teilmengen als Produktgasgemisch A, oder gegebenenfalls nach einer durchgeführten Nebenkomponentenabtrennung als Produktgasgemisch A' der Partialoxidation zuzuführen, während die andere Teilmenge als Bestandteil des Reaktionsgasgemischeingangsgases in die Dehydrierung rückgeführt wird. Der in diesem aus der Dehydrierung selbst kommenden Kreisgas enthaltene molekulare Wasserstoff, soll dann das im Reaktionsgasgemischeingangsgas enthaltene Propan und gegebenenfalls Propylen vor dem in diesem Eingangsgas gleichfalls enthaltenen molekularen Sauerstoff schützen. Dieser Schutz fußt darauf, dass die normalerweise durch die selben Katalysatoren heterogen katalysierte Verbrennung von molekularem Wasserstoff zu Wasser gegenüber der Vollbrennung von Propan und/oder Propylen kinetisch bevorzugt ist.

In der DE-A 102 11 275 wird die Dehydrierkreisgasführung nach dem Strahlpumpenprinzip empfohlen (sie wird auch als Schlaufenfahrweise bezeichnet). Ferner wird in dieser Schrift die Möglichkeit angesprochen, dem Reaktionsgasgemisch in der Propandehydrierung zusätzlich molekularen Wasserstoff zuzusetzen. Über das Erfordernis, den molekularen Wasserstoff in den Treibstrahl für die Strahlpumpe in bestimmter Zufuhrhierarchie zuzudosieren, gibt die DE-A 10 2005 049 699 entsprechende Auskunft.

Die DE-A 10 2004 032 129 und DE-A 10 2005 013 039 schlagen vor, die Rückführung des aus der heterogen katalysierten Partialoxidation herrührenden, molekularen Sauerstoff enthaltenden, Kreisgases 1 nicht in das Reaktionsgasgemischeingangsgas für die heterogen katalysierte partielle Propandehydrierung hinein vorzunehmen. Vielmehr soll die Rückführung erst nach einem bestimmten Dehydrierumsatz in das Reaktionsgasgemisch der Dehydrierung erfolgen. Dabei wird in der DE-A 10 2004 032 129 auch vorgeschlagen, vorab dieser Rückführung dem Reaktionsgasgemisch der Dehydrierung zusätzlich externen molekularen Wasserstoff zuzusetzen. Des weiteren wird auch in der DE-A 10 2004 032 129 die Schlaufenfahrweise für die Dehydrierung propagiert. Treibstrahl ist dabei ausschließlich das aus der Partialoxidation in die Dehydrierung rückgeführte Kreisgas.

Die DE-A 10 2005 009 885 empfiehlt vor diesem Hintergrund im Ausführungsbeispiel 11 eine Schlaufenfahrweise, bei dem das Reaktionsgasgemischeingangsgas für die heterogen katalysierte partielle Dehydrierung des Propans zusammengesetzt ist aus Kreisgas 1, das aus der Partialoxidation rückgeführt wird, aus Frischpropan, aus externem molekularem Wasserstoff, aus einer minimalen Menge an externem Wasserdampf und aus der Dehydrierung selbst rückgeführtem Kreisgas (auf den externen Wasserdampf könnte auch verzichtet werden). Als Treibstrahl wird ein Gemisch aus Frischpropan, externem molekularem Wasserstoff, Kreisgas 1 aus der Partialoxidation und externem Wasserdampf verwendet. Hinsichtlich einer einzuhaltenden Zudosierungsabfolge bei der Treibstrahlerzeugung schweigt sich die DE-A 10 2005 009 885 aus. Diesbezügliche Empfehlungen gibt die DE-A 10 2005 049 699.

Als Dehydrierkatalysatoren für die Reaktionszone A des erfindungsgemäßen Verfahrens kommen grundsätzlich alle im Stand der Technik bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen. Unter anderem können damit alle Dehydrierkatalysatoren eingesetzt werden, die in der WO 01/96270, der EP-A 731077, der DE-A 10211275, der DE-A 10131297, der WO 99/46039, der US-A 4 788 371, der EP-A-0 705 136, der WO 99/29420, der US-A 4 220 091, der US-A 5 430 220, der US-A 5 877 369, der EP-A-0 117 146, der DE-A 199 37 196, der DE-A 199 37 105, der US-A 3,670,044, der US-A 6,566,573, der US-A 4,788,371, der WO-A 94/29021 sowie der DE-A 199 37 1.07 empfohlen werden. Im besonderen können sowohl der Katalysator gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Besonders geeignet ist auch der im Beispiel dieser Schrift eingesetzte Dehydrierkatalysator.

Generell kann es sich bei den Dehydrierkatalysatoren um Katalysatorstränge (Durchmesser typisch 1 bis 10 mm, bevorzugt 1,5 bis 5 mm; Länge typisch 1 bis 20 mm, be- , vorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln. Für eine heterogen katalysierte Dehydrierung im Wirbelbett (bzw. Fließ- oder Wanderbett) wird man entsprechend feinteiligeren Katalysator einsetzen. Erfindungsgemäß bevorzugt ist für die Reaktionszone A das Katalysatorfestbett.

In der Regel sind die Dehydrierkatalysatoren (insbesondere die in dieser Schrift beispielhaft verwendeten sowie die in der DE-A 19937107 empfohlenen (insbesondere die beispielhaften Katalysatoren dieser DE-A) so beschaffen, dass sie sowohl die Dehydrierung von Propan als auch die Verbrennung von Propan und von molekularem Wasserstoff zu katalysieren vermögen. Die Wasserstoffverbrennung verläuft dabei sowohl im Vergleich zur Dehydrierung des Propans als auch im Vergleich zu dessen Verbrennung im Fall einer Konkurrenzsituation an den Katalysatoren sehr viel schneller.

Ferner kommen zur heterogen katalysierten Propandehydrierung in der Reaktionszone A prinzipiell alle im Stand der Technik bekannten Reaktortypen und Verfahrensvarianten in Betracht. Beschreibungen solcher Verfahrensvarianten enthalten zum Beispiel alle bezüglich der Dehydrierkatalysatoren zitierten Schriften des Standes der Technik sowie der eingangs dieser Schrift zitierte Stand der Technik. Eine vergleichsweise ausführliche Beschreibung von für die Reaktionszone A erfindungsgemäß geeigneten Dehydrierverfahren enthält auch Catalytica® Studies Division, Oxidative Dehydrogenation and Alternative Dehydrogenation Processes, Study Number 4192 OD, 1993, 430 Ferguson Drive, Mountain View, California, 94043-5272 U.S.A..

Charakteristisch für die partielle heterogen katalysierte Dehydrierung von Propan ist, dass sie endotherm verläuft. Das heißt, die für die Einstellung der erforderlichen Reaktionstemperatur benötigte Wärme (Energie) muss entweder dem Reaktionsgasausgangsgemisch (dem Reaktionsgasgemischeingangsgas) vorab und/oder im Verlauf der heterogen katalysierten Dehydrierung zugeführt werden.

D.h., bezogen auf den einmaligen Durchgang des der Reaktionszone A zugeführten Beschickungsgasgemischs (Reaktionsgasgemischeingangsgas) durch die Reaktionszone A, kann die Reaktionszone A durch gezielten Wärmeaustausch mit außerhalb der Reaktionszone A geführten (fluiden, d.h., flüssigen oder gasförmigen) Wärmeträgern isotherm gestaltet werden.

Sie kann mit gleicher Bezugsbasis aber auch adibat, d.h., im wesentlichen ohne einen solchen gezielten Wärmeaustausch mit außerhalb der Reaktionszone A geführten Wärmeträgern ausgeführt werden. Im letzteren Fall kann die Bruttowärmetönung, bezogen auf den einmaligen Durchgang des der Reaktionszone A zugeführten Reaktionsgasausgangsgemischs durch die Reaktionszone A, durch Ergreifen von in den vorstehenden (im Stand der Technik gewürdigten) Schriften empfohlenen und im Folgenden noch zu beschreibenden Maßnahmen sowohl endotherm (negativ), oder autotherm (im wesentlichen Null) oder exotherm (positiv) gestaltet werden.

In typischer Weise benötigt die heterogen katalysierte partielle Dehydrierung von Propan zu Propylen vergleichsweise hohe Reaktionstemperaturen. Der dabei erzielbare Umsatz ist normalerweise durch das thermodynamische Gleichgewicht begrenzt. Typische Reaktionstemperaturen betragen 300 bis 800°C bzw. 400 bis 700°C. Pro Molekül an zu Propylen dehydriertem Propan wird dabei ein Molekül Wasserstoff erzeugt.

Hohe Temperaturen und Entfernung des Reaktionsproduktes H₂ begünstigen die Gleichgewichtslage im Sinne des Zielprodukts ebenso wie Partialdruckerniedrigung durch inerte Verdünnung.

Ferner ist es insbesondere für heterogen katalysierte Dehydrierungen von Propan aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberflache abscheiden und selbige dadurch deaktivieren. Um diese nachteilige Begleiterscheinung zu minimieren, kann das zur heterogen katalysierten Dehydrierung bei erhöhter Temperatur über die Katalysatoroberfläche zu leitende Propan haltige Reaktionsgasgemisch mit Wasserdampf verdünnt werden. Sich abscheidender Kohlenstoff wird unter den so gegebenen Bedingungen nach dem Prinzip der Kohlevergasung teilweise oder vollständig eliminiert.

Eine andere Möglichkeit, abgeschiedene Kohlenstoffverbindungen zu beseitigen, besteht darin, den Dehydrierkatalysator von Zeit zu Zeit bei erhöhter Temperatur mit einem Sauerstoff enthaltenden Gas zu durchströmen und damit den abgeschiedenen Kohlenstoff quasi abzubrennen. Eine gewisse Unterdrückung der Bildung von Kohlenstoffablagerungen ist aber auch dadurch möglich, dass man dem heterogen katalysiert zu dehydrierenden Propan, bevor es bei erhöhter Temperatur über den Dehydrierkatalysator geführt wird, molekularen Wasserstoff zusetzt.

Selbstverständlich besteht auch die Möglichkeit, dem heterogen katalysiert zu dehydrierenden Propan Wasserdampf und molekularen Wasserstoff im Gemisch zuzusetzen. Ein Zusatz von molekularem Wasserstoff zur heterogen katalysierten Dehydrierung von Propan mindert auch die unerwünschte Bildung von Allen (Propadien). Propin und Acetylen als Nebenprodukten.

Erfindungsgemäß kann es daher (wie bereits angesprochen) zweckmäßig sein, die Propandehydrierung (z.B. mit vergleichsweise geringem Propanumsatz) (quasi) adiabat durchzuführen. Das heißt, man wird das Reaktionsgasgemischeingangsgas für die Reaktionszone A in der Regel zunächst auf eine Temperatur von 500 bis 700°C (beziehungsweise von 550 bis 650°C) erhitzen (zum Beispiel durch Direktbefeuerung der es umgebenden Wandung). Im Normalfall wird dann ein einziger adiabater Durchgang durch ein Katalysatorbett ausreichend sein, um den gewünschten Umsatz zu erzielen, wobei sich das Reaktionsgasgemisch um etwa 30°C bis 200°C (je nach Umsatz und Verdünnung) abkühlen wird. Ein Beisein von Wasserdampf als Wärmeträger macht sich auch unter dem Gesichtspunkt einer adiabaten Fahrweise vorteilhaft bemerkbar. Die niedrigere Reaktionstemperatur ermöglicht längere Standzeiten des verwendeten Katalysatorbetts.

Prinzipiell ist die heterogen katalysierte Propandehydrierung in der Reaktionszone A (unabhängig ob adiabat oder isotherm gefahren) beim erfindungsgemäßen Verfahren sowohl in einem Festbettreaktor als auch in einem Wanderbett- oder Wirbelbettreaktor durchführbar.

Bemerkenswerterweise ist dabei, insbesondere im adiabatischen Betrieb, ein einzelner Schachtofenreaktor als Festbettreaktor ausreichend, der vom Reaktionsgasgemisch axial und/oder radial durchströmt wird.

Im einfachsten Fall handelt es sich dabei um ein einziges geschlossenes Reaktionsvolumen, zum Beispiel einen Behälter, dessen Innendurchmesser 0,1 bis 10 m, eventuell auch 0,5 bis 5 m beträgt und in welchem das Katalysatorfestbett auf einer Trägervorrichtung (zum Beispiel ein Gitterrost) aufgebracht ist. Das mit Katalysator beschickte Reaktionsvolumen, das im adiabaten Betrieb wärmeisoliert ist, wird dabei mit dem heißen, Propan enthaltenden, Reaktionsgasgemischeingangsgas axial durchströmt. Die Katalysatorgeometrie kann dabei sowohl kugelförmig als auch ringförmig oder strangförmig sein. Da in diesem Fall das Reaktionsvolumen durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Monolithe bzw. Wabenkörper. Zur Verwirklichung einer radialen Strömung des Propan enthaltenden Reaktionsgasgemischs kann der Reaktor zum Beispiel aus zwei in einer Mantelhülle befindlichen, konzentrisch ineinander gestellten, zylindrischen Gitterrosten bestehen und die Katalysatorschüttung in deren Ringspalt angeordnet sein. Im adiabaten Fall wäre die Mantelhülle gegebenenfalls wiederum thermisch isoliert.

Als Katalysatorbeschickung für eine heterogen katalysierte Propandehydrierung mit vergleichsweise geringem Propanumsatz bei einmaligem Durchgang eignen sich insbesondere die in der DE-A 199 37 107 offenbarten, vor allem alle beispielhaft offenbarten, Katalysatoren sowie deren Gemische mit bezüglich der heterogen katalysierten Dehydrierung inerten geometrischen Formkörpern.

Nach längerer Betriebsdauer sind vorgenannte Katalysatoren zum Beispiel in einfacher Weise dadurch regenerierbar, dass man bei einer Eintrittstemperatur von 300 bis 600°C, häufig bei 400 bis 550°C, zunächst in ersten Regenerierungsstufen mit Stickstoff und/oder Wasserdampf (bevorzugt) verdünnte Luft über das Katalysatorbett leitet. Die Katalysatorbelastung mit Regeneriergas kann (bezogen auf die regenerierte Katalysatorgesamtmenge) dabei z.B. 50 bis 10000 h⁻¹ und der Sauerstoffgehalt des Regeneriergases 0,5 bis 20 Vol.-% betragen.

In nachfolgenden weiteren Regenerierungsstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (zum Beispiel N₂) zu spülen.

Anschließend ist es in der Regel empfehlenswert, noch mit reinem molekularem Wasserstoff oder mit durch Inertgas (vorzugsweise Wasserdampf und/oder Stickstoff) verdünntem molekularem Wasserstoff (der Wasserstoffgehalt sollte ≥ 1 Vol.-% betragen) im ansonsten gleichen Bedingungsraster zu regenerieren.

Die heterogen katalysierte Propandehydrierung in der Reaktionszone A des erfindungsgemäßen Verfahrens kann mit vergleichsweise geringem Propanumsatz (≤ 30 mol-%) in allen Fällen bei den gleichen Katalysatorbelastungen (bezogen auf die eingesetzte Katalysatorgesamtmenge) (sowohl das Reaktionsgas insgesamt, als auch das in selbigem enthaltende Propan betreffend) betrieben werden wie bei hohem Propanumsatz (> 30 mol-%). Diese Belastung mit Reaktionsgas kann zum Beispiel 100 bis 10000 h⁻¹, häufig 300 bis 5000 h⁻¹, das heißt vielfach etwa 500 bis 3000 h⁻¹ betragen.

In besonders eleganter Weise lässt sich die heterogen katalysierte Propandehydrierung (vor allem bei geringem Propanumsatz) in der Reaktionszone A (wie bereits angesprochen) in einem Hordenreaktor verwirklichen.

Dieser enthält räumlich aufeinanderfolgend mehr als ein die Dehydrierung katalysierendes Katalysatorbett. Die Katalysatorbettanzahl kann z. B. 1 bis 20, zweckmäßig 2 bis 8, aber auch 3 bis 6 betragen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalte in Segmenten übereinander anzuordnen und das Gas nach radialem Durchtritt in einem Segment in das nächste darüber- oder darunterliegende Segment zu führen.

In zweckmäßiger Weise wird das Reaktionsgasgemisch auf seinem Weg von einem Katalysatorbett zum nächsten Katalysatorbett, zum Beispiel durch Überleiten über mit heißen Gasen erhitzte Wärmetauscherrippen oder durch Leiten durch mit heißen Brenngasen erhitzte Rohre, im Hordenreaktor einer Zwischenerhitzung unterworfen.

Wird der Hordenreaktor im übrigen adiabat betrieben, ist es für Propanumsätze < 30 mol.-% insbesondere bei Verwendung der in der DE-A 199 37 107 beschriebenen Katalysatoren, insbesondere der beispielhaften Ausführungsformen, ausreichend, das Reaktionsgasgemischeingangsgas auf eine Temperatur von 450 bis 550°C (vorzugsweise 450 bis 500°C) vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten zwischen zwei Regenerierungen als besonders günstig erweist.

Noch geschickter ist es, die katalytische Dehydrierung in der Reaktionszone A (wie ebenfalls bereits angesprochen) autotherm, d.h., z.B. die vorstehend geschilderte Zwischenerhitzung auf direktem Weg durchzuführen (autotherme Fahrweise). Dazu wird dem Reaktionsgasgemisch nach Durchströmung des ersten Katalysatorbettes zwischen den nachfolgenden Katalysatorbetten vorteilhaft in begrenztem Umfang zusätzlicher molekularer Sauerstoff zugesetzt. Je nach verwendetem Dehydrierkatalysator wird so eine begrenzte Verbrennung der im Reaktionsgasgemisch enthaltenen Kohlenwasserstoffe, gegebenenfalls bereits auf der Katalysatoroberfläche abgeschiedener Kohle beziehungsweise kohleähnlicher Verbindungen und/oder von im Verlauf der heterogen katalysierten Propandehydrierung gebildetem und/oder dem Reaktionsgasgemisch zugesetztem Wasserstoff bewirkt (es kann auch anwendungstechnisch zweckmäßig sein, im Hordenreaktor Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen zum Beispiel jene der Schriften US-A 4,788,371, US-A 4,886,928, US-A 5,430,209, US-A 5,530,171, US-A 5,527,979 und US-A 5,563,314 in Betracht; beispielsweise können solche Katalysatorbetten in alternierender Weise zu den Dehydrierkatalysator enthaltenden Betten im Hordenreaktor untergebracht sein). Die dabei freigesetzte Reaktionswärme ermöglicht so (quasi adiabate Reaktorgestaltung) auf quasi autotherme Weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender oder im wesentlichen konstanter Temperatur möglich, was besonders lange Standzeiten zwischen zwei Regenerierungen ermöglicht.

In der Regel sollte eine wie vorstehend beschriebene Sauerstoffeinspeisung so vorgenommen werden, dass der Sauerstoffgehalt des Reaktionsgasgemisches, bezogen auf die darin enthaltene Menge an Propan und Propylen, 0,01 bzw.. 0,5 bis 30 Vol.-% beträgt. Als Sauerstoffquelle kommen dabei sowohl reiner molekularer Sauerstoff oder mit Inertgas, zum Beispiel CO, CO₂, N₂, Edelgase, verdünnter Sauerstoff, insbesondere aber auch Luft, in Betracht. Die resultierenden Verbrennungsgase wirken in der Regel zusätzlich verdünnend und fördern dadurch die heterogen katalysierte Propandehydrierung.

Die Isothermie der heterogen katalysierten Propandehydrierung lässt sich dadurch weiter verbessern, dass man im Hordenreaktor in den Räumen zwischen den Katalysatorbetten geschlossene, vor ihrer Füllung günstigerweise aber nicht notwendigerweise evakuierte, Einbauten (zum Beispiel rohrförmige), anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit, das Reaktionsgasgemischeingangsgas für die heterogen katalysierte Propandehydrierung in der Reaktionszone A auf die benötigte Reaktionstemperatur zu erwärmen, besteht auch darin, einen Teil des darin enthaltenen Propans und/oder H₂ mittels im Reaktionsgasgemischeingangsgas enthaltenem molekularem Sauerstoff beim Eintritt in die Reaktionszone A zu verbrennen (zum Beispiel an geeigneten spezifisch wirkenden Verbrennungskatalysatoren, zum Beispiel durch einfaches Überleiten und/oder Durchleiten) und mittels der so freigesetzten Verbrennungswärme die Erwärmung auf die (für die Dehydrierung) gewünschte Reaktionstemperatur zu bewirken. Die resultierenden Verbrennungsprodukte, wie CO₂, H₂O sowie das den für die Verbrennung benötigten molekularen Sauerstoff gegebenenfalls begleitende N₂ bilden vorteilhaft inerte Verdünnungsgase.

Besonders elegant lässt sich die vorgenannte Wasserstoffverbrennung wie in der DE-A 102 11 275 beschrieben realisieren. D.h., in einem Verfahren der kontinuierlichen heterogen katalysierten partiellen Dehydrierung von Propan in der Reaktionszone A, bei dem
- der Reaktionszone A ein molekularen Sauerstoff enthaltendes Kreisgas 1 und Frischpropan sowie molekularen Wasserstoff und gegebenenfalls Wasserdampf enthaltendes Reaktionsgasgemischeingangsgas kontinuierlich zugeführt wird,
- das Reaktionsgasgemischeingangsgas in der Reaktionszone A über wenigstens ein Katalysatorfestbett geführt wird, an welchem durch katalytische Dehydrierung molekularer Wasserstoff und wenigstens partiell Propylen gebildet werden,
- dem Reaktionsgasgemischeingangsgas nach Eintritt in die Reaktionszone A gegebenenfalls weiteres molekularen Sauerstoff enthaltendes Gas zugesetzt wird,
- der molekulare Sauerstoff in der Reaktionszone A im Reaktionsgasgemisch enthaltenen molekularen Wasserstoff teilweise zu Wasserdampf oxidiert und
- der Reaktionszone A ein Produktgasgemisch A entnommen wird, das molekularen Wasserstoff, Wasserdampf, Propylen und Propan enthält,
das dadurch gekennzeichnet ist, dass das der Reaktionszone A entnommene Produktgasgemisch A in zwei Teilmengen identischer Zusammensetzung aufgeteilt und eine der beiden Teilmengen als Kreisgas 2 in das Reaktionsgasgemischeingangsgas für die Reaktionszone A zurückgeführt und die andere Teilmenge erfindungsgemäß als Produktgasgemisch A weiterverwendet wird.

Erfindungsgemäß vorteilhaft wird beim erfindungsgemäßen Verfahren der Reaktionszone A ein Reaktionsgasgemischeingangsgasstrom zugeführt, dessen wesentliche Gehalte typisch wie folgt betragen:

| | |
|---|---|
| Propen | ≥ 0 bis 10, häufig 0 bis 6 Vol.-%; |
| Acrolein | 0 bis 1, vielfach 0 bis 0,5, häufig 0 bis 0,25 Vol.-%; |
| Acrylsäure | 0 bis 0,25, vielfach 0 bis 0,05, häufig 0 bis 0,03 Vol.-%; |
| COₓ | 0 bis 5, vielfach 0 bis 3, häufig 0 bis 2 Vol.-%; |
| Propan | 5 bis 50, vorzugsweise 10 bis 20 Vol.-%; |
| Stickstoff | 30 bis 80, vorzugsweise 50 bis 70 Vol.-%; |
| Sauerstoff | > 0 bis 5, vorzugsweise 1,0 bis 2,0 Vol.-%; |
| H₂O | ≥ 0 bis 20, vorzugsweise 5,0 bis 10,0 Vol.-%; |
| H₂ | 0,5 bis 10, vorzugsweise 1 bis 5 Vol.-%. |

In geringen Mengen (etwa vergleichbar den möglichen Acrylsäuregehalten) kann auch Essigsäure enthalten sein.

Während die EP-A 117 146, die DE-A 22 13 573 und die US-A 3,161,670 empfehlen, Produktgasgemisch A zur Beschickung des wenigstens einen Oxidationsreaktors in der Reaktionszone B zu verwenden, wird es für das erfindungsgemäße Verfahren als vorteilhaft erachtet, aus dem Produktgasgemisch A vor dessen Verwendung zur Erzeugung des Beschickungsgasgemischs für den wenigstens einen Oxidationsreaktor darin enthaltene, von Propan und Propylen verschiedene, Bestandteile wenigstens teilweise abzutrennen. Letzteres kann z. B. dadurch erfolgen, dass man Produktgasgemisch A, gegebenenfalls nachdem man es zuvor in einem indirekten Wärmetauscher abgekühlt hat, über eine, in der Regel zu einem Rohr gestaltete, Membran leitet, die lediglich für molekularen Wasserstoff durchlässig ist. Der so abgetrennte molekulare Wasserstoff kann bei Bedarf teilweise in die Reaktionszone A (z. B. als Bestandteil des Reaktionsgasgemischeingangsgases) zur vorteilhaften Gestaltung der heterogen katalysierten Dehydrierung des Propan rückgeführt oder einer sonstigen Verwertung zugeführt werden. Zusätzlich oder alternativ kann eine Teil- oder die Gesamtmenge von im Produktgasgemisch A üblicherweise enthaltenem Wasserdampf vorab der Verwendung von Produktgasgemisch A zur Beschickung des Oxidationsreaktors in der Reaktionszone B aus selbigem abgetrennt werden (z. B. kondensativ).

Erfindungsgemäß vorteilhaft wird man jedoch wenigstens 50 Vol.-%, vorzugsweise wenigstens 75 Vol.-%, besonders bevorzugt wenigstens 90 Vol.-% und ganz besonders bevorzugt wenigstens 95 Vol.-% der im Produktgasgemisch A enthaltenen, von Propan und Propylen verschiedenen, Bestandteile abtrennen, bevor selbiger als Propenquelle für die Partialoxidation in der Reaktionszone B des erfindungsgemäßen Verfahrens eingesetzt wird. Grundsätzlich können dazu alle in den Schriften DE-A 10 2004 032 129, DE-A 10 2005 013 039, DE-A 10 2005 009 891, DE-A 10 2005 010 111, DE-A 10 2005 009 885, DE-A 10 2005 022 798 und DE-A 10 245 585 beschriebenen Abtrennvarianten angewendet werden.

Eine für die erfindungsgemäßen Bedürfnisse zweckmäßige Möglichkeit dazu besteht z.B. darin, das vorzugsweise abgekühlte (vorzugsweise auf Temperaturen von 10 bis 100 bzw. 70°C) Produktgasgemisch A, z. B. bei einem Druck von 0,1 bis 50 bar, vorzugsweise 5 bis 15 bar und einer Temperatur von z. B. 0 bis 100°C, vorzugsweise 20 bis 40°C, mit einem (vorzugsweise hochsiedenden) organischen Lösungsmittel (vorzugsweise ein hydrophobes), in welchem Propan und Propylen (gegenüber den anderen Bestandteilen des Produktgasgemischs A zweckmäßig bevorzugt) absorbiert werden, in Kontakt zu bringen (z. B. durch einfaches Durchleiten). Durch nachfolgende Desorption, Rektifikation und/oder Strippung mit einem bezüglich der nachfolgenden Propylen-Partialoxidation sich inert verhaltenden und/oder in dieser Partialoxidation als Reaktand benötigten Gas (z. B. Luft oder ein anderes Gemisch aus molekularem Sauerstoff und Inertgas) können das Propan und Propylen im Gemisch in gereinigter Form rückgewonnen und dieses Gemisch für die Partialoxidation als Propylenquelle verwendet werden (vorzugsweise wird wie im Vergleichsbeispiel 1 der deutschen Anmeldung DE-A 10 2004 032 129 beschrieben vorgegangen). Das gegebenenfalls molekularen Wasserstoff enthaltende Abgas einer solchen Absorption kann man z. B. einer Druckwechseladsorptions- und/oder Membrantrennung (z. B. gemäß DE-A 10235419) unterwerfen und dann, den abgetrennten Wasserstoff als Bestandteil des Reaktionsgasgemischeingangsgases für die Reaktionszone A mitverwenden.

Allerdings ist der C3-Kohlenwasserstoffe/C4-Kohlenwasserstoffe Trennfaktor beim vorstehenden Trennverfahren vergleichsweise begrenzt und für die in der DE-A 10245585 beschriebenen Bedürfnisse häufig nicht ausreichend.

Als Alternative für den beschriebenen Trennschritt via Absorption ist für die erfindungsgemäßen Zwecke daher häufig eine Druckwechseladsorption oder eine Druckrektifikation bevorzugt.

Als Absorptionsmittel für die vorstehend beschriebene absorptive Abtrennung eignen sich grundsätzlich alle Absorptionsmittel, die in der Lage sind, Propan und Propylen zu absorbieren. Bei dem Absorptionsmittel handelt es sich vorzugsweise um ein organisches Lösungsmittel, das vorzugsweise hydrophob und/oder hochsiedend ist. Vorteilhafterweise hat dieses Lösungsmittel einen Siedepunkt (bei einem Normaldruck von 1 atm) von mindestens 120°C, bevorzugt von mindestens 180°C, vorzugsweise von 200 bis 350°C, insbesondere von 250 bis 300°C, stärker bevorzugt von 260 bis 290°C. Zweckmäßigerweise liegt der Flammpunkt (bei einem Normaldruck von 1 bar) über 110°C. Allgemein eignen sich als Absorptionsmittel relativ unpolare organische Lösungsmittel, wie zum Beispiel aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und Propylen hat. Beispielhaft als Absorptionsmittel genannt seien aliphatische Kohlenwasserstoffe, zum Beispiel C₈-C₂₀-Alkane oder -Alkene, oder aromatische Kohlenwasserstoffe, zum Beispiel Mittelölfraktionen aus der Paraffindestillation oder Ether mit sperrigen (sterisch anspruchvollen) Gruppen am O-Atom, oder Gemische davon, wobei diesen ein polares Lösungsmittel, wie zum Beispiel das in der DE-A 43 08 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyl-diphenylmethan und dessen Isomere 2-Methyl-2'-benzyl-diphenyl-methan, 2-Methyl-4'-benzyldiphenylmethan und 4-Methyl-2'-benzyl-diphenylmethan und Gemische solcher Isomerer. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, beispielsweise das im Handel erhältliche Diphyl^{®} (z. B. von der Bayer Aktiengesellschaft bezogenes). Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane, Dodecane, Tridecane, Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels ≤ 300 g/mol. Geeignet sind auch die in der DE-A 33 13 573 beschriebenen Paraffinöle mit 8 bis 16 Kohlenstoffatomen. Beispiele für geeignete Handelsprodukte sind von der Firma Haltermann vertriebene Produkte wie Halpasole i, wie Halpasol 250/340 i und Halpasol 250/275 i, sowie Druckfarbenöle unter den Bezeichnungen PKWF und Printosol. Bevorzugt sind an Aromaten freie Handelsprodukte, z. B. solche des Typs PKWFaf. Falls sie einen geringen Restaromatengehalt enthalten, kann dieser vorab des beschriebenen Einsatzes vorteilhaft rektifikativ und/oder adsorptiv gemindert und auf Werte signifikant unter 1000 gew.ppm gedrückt werden. Weitere geeignete Handelsprodukte sind n-Paraffin (C₁₃-C₁₇) oder Mihagol^{®}5 der Erdöl-Raffinerie-Emsland GmbH, LINPAR^{®}14-17 der Firma CONDEA Augusta S.p.A. (Italien) oder der SASOL Italy S.p.A., Normal parrafins (heavy) C₁₄-C₁₈ der Firma SLONAFT in der Slowakei.

Die Gehalte (angegeben in Flächenprozenten der gaschromatographischen Analyse) der vorgenannten Produkte an linearen Kohlenwasserstoffen betragen in typischer Weise:

Summe C₉ bis C₁₃: weniger als 1 %; C₁₄: 30 bis 40 %; C₁₅: 20 bis 33 %; C₁₆: 18 bis 26 %; C₁₇: bis zu 18 %; C≥18: < 2 %.

Eine typische Zusammensetzung des Produktes der Fa. SASOL ist: C₁₃: 0.48 %; C₁₄: 39,8 %; C₁₅: 20,8 %; C₁₆: 18,9 %; C₁₇: 17,3 %; C₁₈: 0,91 %; C₁₉: 0,21 %.

Eine typische Zusammensetzung des Produktes der Fa. Haltermann ist: C₁₃: 0,58 %; C₁₄: 33,4 %; C₁₅: 32,8 %; C₁₆: 25,5 %: C₁₇: 6,8 %; C≥18: < 0,2 %.

Im kontinuierlichen Betrieb wird sich die Zusammensetzung des verwendeten Absorptionsmittels verfahrensbedingt entsprechend ändern.

Die Durchführung der Absorption unterliegt keinen besonderen Beschränkungen. Es können alle dem Fachmann gängigen Verfahren und Bedingungen eingesetzt werden. Vorzugsweise wird der Produktgasgemisch A-Teilstrom 2 bei einem Druck von 1 bis 50 bar, bevorzugt 2 bis 20 bar, stärker bevorzugt 5 bis 15 bar, und einer Temperatur von 0 bis 100°C, insbesondere von 20 bis 50 bzw. 40°C, mit dem Absorptionsmittel in Kontakt gebracht. Die Absorption kann sowohl in Kolonnen als auch in Quenchapparaten vorgenommen werden. Dabei kann im Gleichstrom oder im Gegenstrom (ist bevorzugt) gearbeitet werden. Geeignete Absorptionskolonnen sind zum Beispiel Bodenkolonnen (mit Glocken- und/oder Siebböden), Kolonnen mit strukturierten Packungen (zum Beispiel Blechpackungen mit einer spezifischen Oberfläche von 100 bis 1000, oder bis 750 m²/m³, zum Beispiel Mellapak^{®} 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propans und Propylens von dem Absorptionsmittel kann durch Strippung, Entspannungsverdampfung (Flashen) und/oder Destillation erfolgen.

Die Abtrennung des Propans und Propylens von dem Absorptionsmittel erfolgt vorzugsweise durch Strippen und/oder Desorption. Die Desorption kann in üblicher Weise über eine Druck- und/oder Temperaturänderung durchgeführt werden, vorzugsweise bei einem Druck von 0,1 bis 10 bar, insbesondere 1 bis 5 bar, stärker bevorzugt 1 bis 3 bar, und einer Temperatur von 0 bis 200°C, insbesondere 20 bis 100°C, stärker bevorzugt 30 bis 70°C, besonders bevorzugt 30 bis 50°C. Ein für die Strippung geeignetes Gas ist beispielsweise Wasserdampf, bevorzugt sind jedoch insbesondere Sauerstoff-/Stickstoff-Mischungen, beispielsweise Luft. Bei der Verwendung von Luft beziehungsweise Sauerstoff-/Stickstoff-Mischungen, bei denen der Sauerstoffgehalt über 10 Vol-% liegt, kann es sinnvoll sein, vor und/oder während des Strippprozesses ein Gas zuzusetzen, das den Explosionsbereich reduziert. Besonders geeignet dafür sind Gase mit einer spezifischen Wärmekapazität ≥ 29 J/mol·K bei 20°C, wie zum Beispiel Methan, Ethan, Propan (bevorzugt), Propen, Benzol, Methanol, Ethanol, sowie Ammoniak, Kohlendioxid, und Wasser. C4-Kohlenwasserstoffe sind erfindungsgemäß bevorzugt als solche Zusätze jedoch zu vermeiden. Besonders geeignet für die Strippung sind auch Blasensäulen mit und ohne Einbauten.

Die Abtrennung des Propans und Propylens von dem Absorptionsmittel kann auch über eine Destillation bzw. Rektifikation erfolgen, wobei die dem Fachmann geläufigen Kolonnen mit Packungen, Füllkörpern oder entsprechenden Einbauten verwendet werden können. Bevorzugte Bedingungen bei der Destillation bzw. Rektifikation sind ein Druck von 0,01 bis 5 bar, insbesondere 0,1 bis 4 bar, stärker bevorzugt 1 bis 3 bar, und eine Temperatur (im Sumpf) von 50 bis 300°C, insbesondere 150 bis 250°C.

Eine durch Strippen aus dem Absorptionsmittel gewonnene für die Reaktionszone B des erfindungsgemäßen Verfahrens prinzipiell geeignete Propylenquelle kann vor ihrer Verwendung zur Beschickung der Partialoxidation noch einer weiteren Verfahrensstufe zugeführt werden, um z. B. die Verluste an mitgestripptem Absorptionsmittel zu reduzieren (z. B. Abscheidung in Demistern und/oder Tiefenfiltern) und die erfindungsgemäß durchzuführende Partialoxidation so gleichzeitig vor Absorptionsmittel zu schützen oder um die Trennwirkung zwischen C3-/C4-Kohlenwasserstoffen weiter zu verbessern. Eine solche Abtrennung des Absorptionsmittels kann nach allen dem Fachmann bekannten Verfahrensschritten erfolgen. Eine im Rahmen des erfindungsgemäßen Verfahrens bevorzugte Ausführungsform einer solchen Abtrennung ist z. B. das Quenchen des Ausgangsstromes aus der Strippvorrichtung mit Wasser. In diesem Fall wird das Absorptionsmittel aus diesem beladenen Ausgangsstrom mit Wasser herausgewaschen und der Ausgangsstrom gleichzeitig mit Wasser beladen (geringe Wassermengen wirken sich förderlich auf die Aktivität der Katalysatoren für die erfindungsgemäße Partialoxidation aus). Diese Wäsche beziehungsweise das Quenchen kann z. B. am Kopf einer Desorptionskolonne über einem Flüssigkeits-Fangboden durch Gegensprühen von Wasser oder in einem eigenen Apparat erfolgen.

Zur Unterstützung des Trenneffektes können im Quenchraum die Quenchoberfläche vergößernde Einbauten installiert werden, wie sie dem Fachmann von Rektifikationen, Absorptionen und Desorptionen her bekannt sind.

Wasser ist insofern ein bevorzugtes Waschmittel, da es in der nachfolgenden Partialoxidation normalerweise nicht wesentlich stört. Nachdem das Wasser das Absorptionsmittel aus dem mit Propan und Propylen beladenen Ausgangstrom herausgewaschen hat, kann das wasser/Absorptionsmittel-Gemisch einer Phasentrennung zugeführt und der behandelte, volumenarme, Ausgangstrom, unmittelbar der erfindungsgemäß durchzuführenden Partialoxidation zugeführt werden.

In für das erfindungsgemäße Verfahren vorteilhafter Weise lassen sich insbesondere dann, wenn das Propylen/Propan-Gemisch aus dem Absorbat mittels Luft freigestrippt wird, in der Regel unmittelbar für die Partialoxidation verwendbare Reaktionsgasausgangsgemische gewinnen. Für den Fall, dass deren Propangehalt erfindungsgemäß noch nicht befriedigen sollte, kann ihnen vor ihrer Verwendung für die erfindungsgemäß durchzuführende Partialoxidation des enthaltenen Propylens noch Frischpropan zugesetzt werden. Über das Restgas (Kreisgas 1) wird selbiges dann zweckmäßig in die heterogen katalysierte Dehydrierung (vorteilhaft als Bestandteil des in die Reaktionszone A geführten Reaktionsgasgemischeingangsgasstroms) rückgeführt werden. Um die entsprechende Propanmenge kann dann die direkte Zufuhr an Frischpropan in die Reaktionszone A gemindert werden. Im Extremfall kann die für die heterogen katalysierte Propandehydrierung erforderliche Zufuhr an Frischpropan unmittelbar in die Reaktionszone A hinein beim erfindungsgemäßen Verfahren dann vollständig entfallen, wenn diese Zufuhr an Frischpropan vor der Durchführung der Partialoxidation des Propylens vollständig ins diesbezügliche Reaktionsgasausgangsgemisch hinein erfolgt, von wo aus es dann als verbleibender Bestandteil im Restgas (Kreisgas 1) erst nach Durchlaufen der erfindungsgemäß durchzuführenden Partialoxidation als Bestandteil des Reaktionsgasgemischeingangsgasstroms für die heterogen katalysierte Propandehydrierung der Reaktionszone A zugeführt wird. Gegebenenfalls kann beim erfindungsgemäßen Verfahren eine Frischpropanzufuhr auch in eine gegebenenfalls zwischen heterogen katalysierte Dehydrierung und Propylenpartialoxidation befindliche C₃-Abtrennung hinein (z. B. als Stripgas) erfolgen.

Handelt es sich um eine zweistufige Partialoxidation von Propylen zu Acrylsäure, kann ein Teil oder auch die vollständige Zufuhr an Frischpropan auch in das Reaktionsgasausgangsgemisch für die zweite Stufe der Partialoxidation hinein erfolgen (allerdings ist dieses Reaktionsgasausgangsgemisch manchmal bereits dann nicht explosiv, wenn diese Qualifizierung schon auf das Reaktionsgasausgangsgemisch für die erste Stufe der Partialoxidation zutraf). Dies ist vor allem deshalb vorteilhaft, weil die unerwünschte Nebenreaktion von Propan zu Propionaldehyd und/oder Propionsäure vor allem von der ersten Partialoxidationsstufe (Propylen - Acrolein) unter deren Bedingungen ausgeht. Vorteilhaft ist es auch, eine Frischpropanzufuhr auf die erste und auf die zweite Partialoxidationsstufe im wesentlichen gleichmäßig zu verteilen.

Durch diese Möglichkeit der Zufuhr von Frischpropan ins Reaktionsgasausgangsgemisch für die Partialoxidationsstufen hinein, lässt sich die Zusammensetzung dieser Reaktionsgasausgangsgemische zielsicher nicht explosiv gestalten. Gegebenenfalls kann auch eine Teilmenge an Restgas zu diesem Zweck direkt in die Propylen- und/oder Acroleinpartialoxidation rückgeführt werden. Bei Bedarf kann auch ein Gemisch aus Frischpropan und Restgas für diesen Zweck verwendet werden. Entscheidend für die Beantwortung der Frage, ob das Reaktionsgasausgangsgemisch für eine Partialoxidationsstufe explosiv ist oder nicht, ist, ob sich in dem unter bestimmten Ausgangsbedingungen (Druck, Temperatur) befindlichen Reaktionsgasausgangsgemisch eine durch eine örtliche Zündquelle (z. B. glühender Platindraht) eingeleitete Verbrennung (Entzündung, Explosion) ausbreitet oder nicht (vgl. DIN 51649 und die Untersuchungsbeschreibung in der WO 04/007405). Erfolgt eine Ausbreitung, soll das Gemisch hier als explosiv bezeichnet werden. Erfolgt keine Ausbreitung, wird das Gemisch in dieser Schrift als nicht explosiv eingeordnet. Ist das Reaktionsgasausgangsgemisch nicht explosiv, gilt dies auch für die im Verlauf der erfindungsgemäßen Partialoxidation des Propylens gebildeten Reaktionsgasgemische (vgl. WO 04/007405).

In der Regel wird beim erfindungsgemäßen Verfahren jedoch nur der der Reaktionszone A zugeführte Reaktionsgasgemischeingangsgasstrom Frischpropan zugesetzt enthalten.

Vorliegende Erfindung betrifft aber auch Verfahrensausgestaltungen, bei denen man das für das Verfahren benötigte Frischpropan höchstens teilweise (z. B. nur zu 75 %, oder nur zu 50 %, oder nur zu 25 %) diesem Reaktionsgasgemischeingangsstrom und wenigstens teilweise (in der Regel die Restmenge, gegebenenfalls die Gesamtmenge) dem bzw. den Reaktionsgasgemischausgangsgasen der Partialoxidation zuführt. Im übrigen kann wie in der WO 01196170 beschrieben verfahren werden, die einen integralen Bestandteil dieser Anmeldung bildet.

In an sich bekannter Weise läuft die heterogen katalysierte Gasphasen-Partialoxidation von Propylen zu Acrylsäure mit molekularem Sauerstoff grundsätzlich in zwei längs der Reaktionskoordinate aufeinanderfolgenden Schritten ab, von denen der erste zum Acrolein und der zweite von Acrolein zu Acrylsäure führt.

Dieser Reaktionsablauf in zwei zeitlich aufeinanderfolgenden Schritten eröffnet in an sich bekannter Weise die Möglichkeit, das erfindungsgemäße Verfahren auf der Stufe des Acrolein (der Stufe überwiegender Acroleinbildung) abzubrechen und die Zielproduktabtrennung auf dieser Stufe vorzunehmen, oder das erfindungsgemäße Verfahren bis zur überwiegenden Acrylsäurebildung fortzuführen und die Zielproduktabtrennung erst dann vorzunehmen.

Führt man das erfindungsgemäße Verfahren bis zur überwiegenden Acrylsäurebildung, ist es erfindungsgemäß vorteilhaft, das Verfahren zweistufig, d. h. in zwei hintereinander angeordneten Oxidationsstufen auszuführen, wobei zweckmäßigerweise in jeder der beiden Oxidationsstufen das zu verwendende Katalysatorfestbett und vorzugsweise auch die sonstigen Reaktionsbedingungen, wie z. B. die Temperatur des Katalysatorfestbetts, in optimierender Weise angepasst werden.

Zwar vermögen die für die Katalysatoren der ersten Oxidationsstufe (Propylen -> Acrolein) als Aktivmassen besonders geeigneten, die Elemente Mo, Fe, Bi enthaltenden Multimetalloxide, in gewissem Umfang auch die zweite Oxidationsstufe (Acrolein -> Acrylsäure) zu katalysieren, doch werden für die zweite Oxidationsstufe normalerweise Katalysatoren bevorzugt, deren Aktivmasse wenigstens ein die Elemente Mo und V enthaltendes Multimetalloxid ist.

Damit eignet sich das erfindungsgemäß durchzuführende Verfahren der heterogen katalysierten Partialoxidation von Propylen an Katalysatorfestbetten, deren Katalysatoren als Aktivmasse wenigstens ein die Elemente Mo, Fe und Bi enthaltendes Multimetalloxid aufweisen, insbesondere als einstufiges Verfahren zur Herstellung von Acrolein (sowie gegebenenfalls Acrylsäure) oder als erste Reaktionsstufe zur zweistufigen Her- , stellung von Acrylsäure.

Die Realisierung der einstufigen heterogen katalysierten Partialoxidation von Propylen zu Acrolein sowie gegebenenfalls Acrylsäure bzw. der zweistufigen heterogen katalysierten Partialoxidation von Propylen zu Acrylsäure unter Verwendung eines erfindungsgemäß erzeugten Reaktionsgasausgangsgemischs kann dabei im einzelnen wie in den Schriften EP-A 70 07 14 (erste Reaktionsstufe; wie dort beschrieben, aber auch in entsprechender Gegenstromfahrweise von Salzbad und Reaktionsgasausgangsgemisch über den Rohrbündelreaktor), EP-A 70 08 93 (zweite Reaktionsstufe; wie dort beschrieben, aber auch in entsprechender Gegenstromfahrweise), WO 04/085369 (insbesondere diese Schrift wird als integraler Bestandteil dieser Schrift betrachtet) (als zweistufiges Verfahren), WO 04/85363, DE-A 103 13 212 (erste Reaktionsstufe), EP-A 1 159 248 (als zweistufiges Verfahren), EP-A 1 159 246 (zweite Reaktionsstufe), EP-A 1 159 247 (als zweistufiges Verfahren), DE-A 199 48 248 (als zweistufiges Verfahren), DE-A 101 01 695 (einstufig oder zweistufig), WO 04/085368 (als zweistufiges Verfahren), DE 10 2004 021 764 (zweistufig), WO 04/085362 (erste Reaktionsstufe), WO 04/085370 (zweite Reaktionsstufe), WO 04/085365 (zweite Reaktionsstufe), WO 04/085367 (zweistufig), EP-A 990 636, EP-A 1 007 007 und EP-A 1 106 598 beschrieben durchgeführt werden.

Dies gilt insbesondere für alle in diesen Schriften enthaltenen Ausführungsbeispiele. Sie können wie in diesen Schriften beschrieben durchgeführt werden, jedoch mit dem Unterschied, dass als Reaktionsgasausgangsgemisch für die erste Reaktionsstufe (Propylen zu Acrolein) ein gemäß der vorliegenden Erfindung erzeugtes Reaktionsgasausgangsgemisch eingesetzt wird. Die übrigen Parameter betreffend wird wie in den Ausführungsbeispielen der genannten Schriften verfahren (insbesondere die Katalysatorfestbetten und Reaktandenbelastung der Katalysatorfestbetten betreffend). Wird in den vorgenannten Ausführungsbeispielen des Standes der Technik zweistufig verfahren und erfolgt zwischen den beiden Reaktionsstufen eine Sekundärsauerstoff(Sekundärluft)einspeisung, so wird diese in entsprechender Weise vorgenommen, in ihrer Menge jedoch dahingehend angepasst, dass das molare Verhältnis von molekularem Sauerstoff zu Acrolein im Beschickungsgemisch der zweiten Reaktionsstufe demjenigen in den Ausführungsbeispielen der genannten Schriften entspricht. Ein erfindungsgemäßes Reaktionsgasausgangsgemisch für die heterogen katalysierte partielle Gasphasenoxidation ist z. B. in einfacher Weise dadurch erhältlich, dass man dem Produktgasgemisch A oder dem Produktgasgemisch A' soviel molekularen Sauerstoff zusetzt, wie für die Partialoxidation benötigt wird. Diese Zufuhr kann als reiner molekularer Sauerstoff oder auch als ein Gemisch aus molekularem Sauerstoff und aus Inertgas (oder auch nur im Beisein von Inertgas) erfolgen. Als ein solches Gemisch wird erfindungsgemäß Luft bevorzugt. Erfindungsgemäß wesentlich ist, dass diese Sauerstoffzufuhr so erfolgt, dass das Produktgasgemisch B noch nicht umgesetzten molekularen Sauerstoff enthält.

In der Regel wird das molare Verhältnis von im Reaktionsgasausgangsgemisch für die Partialoxidation enthaltenem molekularem Sauerstoff zu im Reaktionsgasausgangsgemisch für die Partialoxidation enthaltenem Propylen ≥ 1 und ≤ 3 betragen.

Für die jeweilige Reaktionsstufe besonders geeignete Multimetalloxidkatalysatoren sind vielfach vorbeschrieben und dem Fachmann wohlbekannt. Beispielsweise verweist die EP-A 25 34 09 auf Seite 5 auf entsprechende US-Patente.

Günstige Katalysatoren für die jeweilige Oxidationsstufe offenbaren auch die DE-A 4 431 957, die DE-A 10 2004 025 445 und die DE-A 4 431 949. Dieses gilt insbesondere für jene der allgemeinen Formel 1 ihn den beiden vorgenannten älteren Schriften. Besonders vorteilhafte Katalysatoren für die jeweilige Oxidationsstufe offenbaren die Schriften DE-A 103 25 488, DE-A 103 25 487, DE-A 103 53 954, DE-A 103 44 149, DE-A 103 51 269, DE-A 103 50 812 und DE-A 103 50 822.

Für die erfindungsgemäße Reaktionsstufe der heterogen katalysierten Gasphasen-Partialoxidation von Propylen zu Acrolein oder Acrylsäure oder deren Gemisch, kommen prinzipiell alle Mo, Bi und Fe enthaltenden Multimetalloxidmassen als Aktivmasse in Betracht.

Dies sind insbesondere die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, II und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 7 00 714 genannten Multimetalloxidaktivmassen.

Ferner eignen sich für diese Reaktionsstufe die Mo, Bi und Fe enthaltenden Multimetalloxidkatalysatoren die in den Schriften Research Disclosure Nr. 497012 vom 29.08.2005, DE-A 100 46 957, DE-A 100 63 162, DE-C 3 338 380, DE-A 199 02 562, EP-A 15 565, DE-C 2 380 765, EP-A 8 074 65, EP-A 27 93 74, DE-A 330 00 44, EP-A 575897, US-A 4438217, DE-A 19855913, WO 98/24746, DE-A 197 46 210 (diejenigen der allgemeinen Formel II), JP-A 91/294239, EP-A 293 224 und EP-A 700 714 offenbart werden. Dies gilt insbesondere für die beispielhaften Ausführungsformen in diesen Schriften, unter denen jene der EP-A 15565, der EP-A 575897, der DE-A 197 46 210 und der DE-A 198 55 913 besonders bevorzugt werden. Besonders hervorzuheben sind in diesem Zusammenhang ein Katalysator gemäß Beispiel 1 c aus der EP-A 15 565 sowie ein in entsprechender Weise herzustellender Katalysator, dessen Aktivmasse jedoch die Zusammensetzung Mo₁₂Ni_{6,5}Zn₂Fe₂Bi₁P_{0,0065}K_{0,06}Oₓ-10SiO₂ aufweist. Ferner sind hervorzuheben das Beispiel mit der laufenden Nr. 3 aus der DE-A 198 55 913 (Stöchiometrie: Mo₁₂Co₇Fe₃B_{i0,8}K_{0,08}Si_{1,6}Oₓ) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Weitere mögliche Katalysatorgeometrien sind in diesem Zusammenhang Stränge (z. B. 7,7 mm Länge und 7 mm Durchmesser; oder 6,4 mm Länge und 5,7 mm Durchmesser).

Eine Vielzahl der für den Schritt von Propylen zu Acrolein und gegebenenfalls Acrylsäure geeigneten Multimetalloxidaktivmassen lässt sich unter der allgemeinen Formel IV,

Mo₁₂BiₐFe_{b}X¹_{c}X²_{d}X³ₑX⁴_{f}Oₙ (IV),

in der die Variablen nachfolgende Bedeutung aufweisen:
- X¹ =: Nickel und/oder Kobalt,
- X² =: Thallium, ein Alkalimetall und/oder ein Erdalkalimetall,
- X³ =: Zink, Phosphor, Arsen, Bor, Antimon, Zinn, Cer, Blei und/oder Wolfram,
- X⁴ =: Silicium, Aluminium, Titan und/oder Zirkonium,

- a=: 0,5 bis 5,
- b=: 0,01 bis 5, vorzugsweise 2 bis 4,
- c=: 0 bis 10, vorzugsweise 3 bis 10,
- d=: 0 bis 2, vorzugsweise 0,02 bis 2,
- e=: 0 bis 8, vorzugsweise 0 bis 5,
- f=: 0 bis 10 und
- n=: eine Zahl, die durch die Wertigkeit und Häufigkeit der von Sauerstoff verschiedenen Elemente in IV bestimmt wird,
subsumieren.

Sie sind in an sich bekannter Weise erhältlich (siehe z. B. die DE-A 4 023 239) und werden üblicherweise in Substanz zu Kugeln, Ringen oder Zylindern geformt oder auch in Gestalt von Schalenkatalysatoren, d. h., mit der Aktivmasse beschichteten vorgeformten, inerten Trägerkörpern, eingesetzt. Selbstverständlich können sie aber auch in Pulverform als Katalysatoren angewendet werden:

Prinzipiell können Aktivmassen der allgemeinen Formel IV in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH, (NH₄)₂CO_{3,} NH₄NO₃, NH₄CHO₂, CH₃COOH, NH₄CH₃CO₂und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird.

Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Multimetalloxidaktivmassen der allgemeinen Formel IV können für den Schritt "Propylen - Acrolein (und gegebenenfalls Acrylsäure)" sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Anstelle von Graphit kann aber auch hexagonales Bornitrid als Hilfsmittel bei der Formgebung verwendet werden, wie es die DE-A 10 2005 037 678 empfiehlt. Geeignete Volikatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 29 09 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Schritt vom Propylen zum Acrolein (sowie gegebenenfalls Acrylsäure) zu verwendende Multimetalloxidaktivmassen sind ferner Massen der allgemeinen Formel V,

[Y¹ₐ·Y²_{b}·Oₓ·]p[Y³_{c}·Y⁴_{d}·Y⁵ₑ·Y⁶_{f}·Y⁷_{g}·Y²ₕ·O^{y}·]_{q} (V),

in der die Variablen folgende Bedeutung haben:
- Y¹ =: nur Wismut oder Wismut und wenigstens eines der Elemente Tellur, Antimon, Zinn und Kupfer,
- Y² =: Molybdän, oder Wolfram, oder Molybdän und Wolfram,
- Y³ =: ein Alkalimetall, Thallium und/oder Samarium,
- Y⁴ =: ein Erdalkalimetall, Nickel, Kobalt, Kupfer, Mangan, Zink, Zinn, Cadmium und/oder Quecksilber,
- Y⁵ =: Eisen oder Eisen und wenigstens eines der Elemente Chrom und Cer,
- Y⁶: Phosphor, Arsen, Bor und/oder Antimon,
- Y⁷ =: ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

- a' =: 0,01 bis 8,
- b' =: 0,1 bis 30,
- c' =: 0 bis 4,
- d' =: 0 bis 20,
- e' >: 0 bis 20,
- f =: 0 bis 6,
- g' =: 0 bis 15,
- h' =: 8 bis 16,
- x',y'=: Zahlen, die durch die Wertigkeit und Häufigkeit der von Sauerstoff verschiedenen Elemente in V bestimmt werden und
- p,q =: Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹ₐ·Y²_{b}·Oₓ·, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte erfindungsgemäß geeignete Multimetalloxidmassen V sind solche, in denen Y¹ nur Wismut ist.

Unter diesen werden wiederum jene bevorzugt, die der allgemeinen Formel VI,

[Bi_{a"}Z²_{b"}O_{x"}]_{p"} [Z²₁₂Z³_{c"}Z⁴_{d"}Fe_{e"}Z⁵_{p"}Z⁶_{g"}Z_{h"}O_{y"}]_{q"} (VI),

in der die Variablen folgende Bedeutung haben:
- Z² =: Molybdän, oder Wolfram, oder Molybdän und Wolfram,
- Z³ =: Nickel und/oder Kobalt,
- Z⁴ =: Thallium, ein Alkalimetall und/oder ein Erdalkalimetall,
- Z⁵ =: Phosphor, Arsen, Bor, Antimon, Zinn, Cer und/oder Blei,
- Z⁶ =: Silicium, Aluminium, Titan und/oder Zirkonium,
- Z⁷ =: Kupfer, Silber und/oder Gold,

- a" =: 0,1 bis 1,
- b" =: 0,2 bis 2,
- c" =: 3 bis 10,
- d" =: 0,02 bis 2,
- e" =: 0,01 bis 5, vorzugsweise 0,1 bis 3,
- f" =: 0 bis 5,
- g" =: 0 bis 10,
- h": = 0 bis 1,
- x",y"=: Zahlen, die durch die Wertigkeit und Häufigkeit der von Sauerstoff verschiedenen Element in VI bestimmt werden,
- p",q"=: Zahlen, deren Verhältnis p"/q" 0,1 bis 5, vorzugsweise 0,5 bis 2 beträgt,
entsprechen, wobei diejenigen Massen VI ganz besonders bevorzugt werden, in denen Z²_{b"} = (Wolfram)_{b"} und Z²₁₂ = (Molybdän)₁₂ ist.

Ferner ist es von Vorteil, wenn wenigstens 25 mol-% (bevorzugt wenigstens 50 mol-% und besonders bevorzugt 100 mol-%) des gesamten Anteils [Y¹_{a'}Y²_{b'}O_{x'}]ₚ ([Biₐ-Z²_{b}-Oₓ-]p-) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung
Y¹_{a'}Y²_{b'}O_{x'} [Bi_{a"}Z²_{b"}O_{x"}] vorliegen, deren Größtdurchmesser im Bereich 1 nm bis 100 µm liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.

Für den zweiten Schritt(die zweite Reaktionsstufe), die heterogen katalysierte Gasphasen-Partialoxidation von Acrolein zu Acrylsäure, kommen, wie bereits gesagt, prinzipiell alle Mo und V enthaltenden Multimetalloxidmassen als Aktivmassen für die benötigten Katalysatoren in Betracht, z. B. jene der DE-A 100 46 928.

Eine Vielzahl derselben, z. B. diejenigen der DE-A 198 15 281, lässt sich unter der allgemeinen Formel VII,

Mo₁₂VₐX¹_{b}X²_{c}X³_{d}X⁴ₑX⁵_{f}X⁶_{g}Oₙ (VII),

in der die Variablen folgende Bedeutung haben:
- X¹ =: W, Nb, Ta, Cr und/oder Ce,
- X² =: Cu, Ni, Co, Fe, Mn und/oder Zn,
- X³ =: Sb und/oder Bi,
- X⁴ =: eines oder mehrere Alkalimetalle,
- X⁵ =: eines oder mehrere Erdalkalimetalle,
- X⁶ =: Si, Al, Ti und/oder Zr,

- a =: 1 bis 6,
- b =: 0,2 bis 4,
- c =: 0,5 bis 18,
- d =: 0 bis 40,
- e =: 0 bis 2,
- f =: 0 bis 4,
- g =: 0 bis 40 und
- n =: eine Zahl, die durch die Wertigkeit und Häufigkeit der von Sauerstoff verschiedenen Elemente in VII bestimmt wird,
subsumieren.

Erfindungsgemäß bevorzugte Ausführungsformen innerhalb der aktiven Multimetalloxide VII sind jene, die von den nachfolgenden Bedeutungen der Variablen der allgemeinen Formel VII erfasst werden:
- X¹ =: W, Nb, und/oder Cr,
- X² =: Cu, Ni, Co, und/oder Fe,
- X³ =: Sb,
- X⁴ =: Na und/oder K,
- X⁵ =: Ca, Sr und/oder Ba,
- X⁶ =: Si, Al, und/oder Ti,

- a =: 1,5 bis 5,
- b =: 0,5 bis 2,
- c =: 0,5 bis 3,
- d =: 0 bis 2,
- e =: 0 bis 0,2,
- f =: 0 bis 1 und
- n =: eine Zahl, die durch die Wertigkeit und Häufigkeit der von Sauerstoff verschiedenen Elemente in VII bestimmt wird.

Erfindungsgemäß ganz besonders bevorzugte Multimetalloxide VII sind jedoch jene der allgemeinen Formel VIII,

Mo₁₂Vₐ·Y¹_{b}·Y²_{c}·Y⁵_{f}·Y⁶_{g}· Oₙ₋ (VIII),

mit
- Y¹ =: W und/oder Nb,
- Y² =: Cu und/oder Ni,
- Y⁵ =: Ca und/oder Sr,
- Y⁶ =: Si und/oder Al,
- a' =: 2 bis 4,
- b' =: 1 bis 1,5,
- c' =: 1 bis 3,
- f =: 0 bis 0,5
- g' =: 0 bis 8 und
- n' =: eine Zahl, die durch die Wertigkeit und Häufigkeit der von Sauerstoff verschiedenen Elementen in VIII bestimmt wird.

Die erfindungsgemäß geeigneten Multimetalloxidaktivmassen (VII) sind in an sich bekannter, z. B. in der DE-A 43 35 973 oder in der EP-A 714 700 offenbarter, Weise erhältlich.

Prinzipiell können für den Schritt "Acrolein → Acrylsäure" geeignete Multimetalloxidaktivmassen, insbesondere solche der allgemeinen Formel VII, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel VII, können für die Acroleinoxidation sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z. B. 8,2 mm oder 5,1 mm) betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberfilächenrauhen, kugelförmigen Trägern aus Steatit, deren Durchmesser 1 bis 10 mm bzw. bis 8 mm, bevorzugt 4 bis 5 mm beträgt. D. h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für den Schritt "Acrolein → Acrylsäure" zu verwendende Multimetalloxidaktivmassen sind ferner Massen der allgemeinen Formel IX,

[D]ₚ[E]_{q} (IX),

in der die Variablen folgende Bedeutung haben:
- D =: Mo₁₂V_{a"}Z¹_{b"}Z²_{c"}Z³_{d"}Z⁴_{e"}Z⁵_{f"}Z⁶_{g"}O_{x"},
- E =: Z⁷₁₂Cuₕ-Hᵢ-O_{y"},
- Z¹ =: W, Nb, Ta, Cr und/oder Ce,
- Z² =: Cu, Ni, Co, Fe, Mn und/oder Zn,
- Z³ =: Sb und/oder Bi,
- Z⁴ =: Li, Na, K, Rb, Cs und/oder H
- Z⁵ =: Mg, Ca, Sr und/oder Ba,
- Z⁶ =: Si, Al, Ti und/oder Zr,
- Z⁷ =: Mo, W, V, Nb und/oder Ta, vorzugsweise Mo und/oder W

- a" =: 1 bis 8,
- b" =: 0,2 bis 5,
- c" =: 0 bis 23,
- d" =: 0 bis 50,
- e" =: 0 bis 2,
- f" =: 0 bis 5,
- g" =: 0 bis 50,
- h" =: 4 bis 30,
- i" =: 0 bis 20 und
- x",y" =: Zahlen, die durch die Wertigkeit und Häufigkeit der von Sauerstoff verschiedenen Element in IX bestimmt werden und
- p,q =: von Null verschiedene Zahlen, deren Verhältnis p/q 160:1 bis 1:1 beträgt,
und die dadurch erhältlich sind, dass man eine Multimetalloxidmasse E

Z⁷₁₂Cu_{h"}H_{i"}O_{y"} (E),

in feinteiliger Form getrennt vorbildet (Ausgangsmasse 1) und anschließend die vorgebildete feste Ausgangsmasse 1 in eine wäßrige Lösung, eine wäßrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D

Mo₁₂V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z. B. die EP-A 668 104, die DE-A 197 36 105, die DE-A 100 46 928, die DE-A 197 40 493 und die DE-A 195 28 646.

Hinsichtlich der Formgebung gilt bezüglich Multimetalloxidmassen IX-Katalysatoren das bei den Multimetalloxidmassen VII-Katalysatoren Gesagte.

Für den Schritt "Acrolein - Acrylsäure" in hervorragender Weise geeignete Multimetalloxidkatalysatoren sind auch jene der DE-A 198 15 281, insbesondere mit Multimetalloxidaktivmassen der allgemeinen Formel I dieser Schrift.

Vorteilhaft werden für den Schritt vom Propylen zum Acrolein Vollkatalysatorringe und für den Schritt vom Acrolein zur Acrylsäure Schalenkatalysatorringe verwendet.

Die Durchführung der Paritaloxidation des erfindungsgemäßen Verfahrens, vom Propylen zum Acrolein (sowie gegebenenfalls Acrylsäure), kann mit den beschriebenen Katalysatoren z. B. in einem Einzonen-Vielkontaktrohr-Festbettreaktor durchgeführt werden, wie ihn die DE-A 4 431 957 beschreibt. Dabei können Reaktionsgasgemisch und Wärmeträger (Wärmeaustauschmittel) über den Reaktor betrachtet im Gleichstrom oder im Gegenstrom geführt werden.

Der Reaktionsdruck liegt üblicherweise im Bereich von 1 bis 3 bar und die Gesamtraumbelastung des Katalysatorfestbetts mit Reaktionsgas(ausgangs)gemisch 2 beträgt vorzugsweise 1500 bis 4000 bzw. 6000 Nl/l•h oder mehr. Die Propylenlast (die Propylenbelastung des Katalysatorfestbetts) beträgt typisch 90 bis 200 Nl/l•h oder bis 300 Nl/l•h oder mehr. Propylenlasten oberhalb von 135 Nl/l•h bzw. ≥ 140 Nl/l•h, oder ≥ 150 Nl/l•h, oder ≥ 160 Nl/l•h sind erfindungsgemäß besonders bevorzugt, da das erfindungsgemäße Reaktionsgasausgangsgemisch infolge des Beiseins von nicht umgesetztem Propan ein günstiges Heißpunktverhalten bedingt (alles Vorgenannte gilt auch unabhängig von der speziellen Wahl des Festbettreaktors).

Bevorzugt wird der Einzonen-Vielkontaktrohr-Festbettreaktor mit dem Beschickungsgasgemisch von oben angeströmt. Als Wärmeaustauschmittel wird zweckmäßig eine Salzschmelze, bevorzugt bestehend aus 60 Gew.-% Kaliumnitrat (KNO₃) und 40 Gew.-% Natriumnitrit (NaNO₂), oder aus 53 Gew.-% Kaliumnitrat (KNO₃), 40 Gew.-% Natriumnitrit (NaNO₂) und 7 Gew.-% Natriumnitrat (NaNO₃), eingesetzt.

Über den Reaktor betrachtet können, wie bereits gesagt, Salzschmelze und Reaktionsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt mit Katalysator zu beschicken (für die Anströmung von unten nach oben wird die Beschickungsabfolge in zweckmäßiger Weise umgekehrt):
- zunächst auf einer Länge von 40 bis 80 bzw. bis 60 % der Kontaktrohrlänge entweder nur Katalysator, oder ein Gemisch aus Katalysator und Inertmaterial, wobei letzteres, bezogen auf die Mischung, einen Gewichtsanteil von bis zu 30 bzw. bis zu 20 Gew.-% ausmacht (Abschnitt C);
- daran anschließend auf einer Länge von 20 bis 50 bzw. bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 40 Gew.-% ausmacht (Abschnitt B); und
- abschließend auf einer Länge von 10 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt.

Vorgenannte Beschickungsvariante ist insbesondere dann zweckmäßig, wenn als Katalysatoren solche gemäß Research Disclosure Nr. 497012 vom 29.08.2005, oder gemäß Beispiel 1 der DE-A 100 46 957 oder gemäß Beispiel 3 der DE-A 100 46 957 und als Inertmaterial Ringe aus Steatit mit der Geometrie 7 mm x 7 mm x 4 mm (Außendurchmesser x Höhe x Innendurchmesser) verwendet werden. Hinsichtlich der Salzbadtemperatur gilt das in der DE-A 4 431 957 Gesagte.

Die Durchführung der erfindungsgemäßen Partialoxidation, vom Propylen zum Acrolein (und gegebenenfalls Acrylsäure), kann mit den beschriebenen Katalysatoren aber auch z. B. in einem Zweizonen-Vielkontaktrohr-Festbettreaktor durchgeführt werden, wie es die DE-A 199 10 506, die DE-A 10 2005 009 885, die DE-A 10 2004 032 129, DE-A 10 2005 013 039 und die DE-A 10 2005 009 891 sowie die DE-A 10 2005 010 111 beschreibt. In beiden vorstehend beschriebenen Fällen (sowie ganz allgemein beim erfindungsgemäßen Verfahren) liegt der erzielte Propenumsatz bei einfachem Durchgang normalerweise bei Werten ≥ 90 mol-%, bzw. ≥ 95 mol-% und die Selektivität der Acroleinbildung bei Werten ≥ 90 mol-%. Erfindungsgemäß vorteilhaft erfolgt die erfindungsgemäße Partialoxidation von Propen zu Acrolein, oder Acrylsäure oder deren Gemisch wie in der EP-A 1159244 und ganz besonders bevorzugt wie in der WO 04/085363 sowie in der WO 04/085362 beschrieben.

Die Schriften EP-A 1159244, WO 04/085363 und WO 04/085362werde als integraler Bestandteil dieser Schrift betrachtet.

D. h., die erfindungsgemäß durchzuführende Partialoxidation des Propylens lässt sich besonders vorteilhaft an einem Katalysatorfestbett mit erhöhter Propylenbelastung durchführen, das wenigstens zwei Temperaturzonen aufweist.

Diesbezüglich wird z. B. auf die EP-A 1159244 und die WO 04/085362 verwiesen.

Eine typische Zusammensetzung des Reaktionsgasausgangsgemischs für die Partialoxidation vom Propylen zur Acrolein kann beim erfindungsgemäßen Verfahren umfassen:

| | |
|---|---|
| 5 bis 9 Vol.-% | Propylen, |
| 8 bis 18 Vol.-% | molekularer Sauerstoff, |
| 6 bis 30 (bzw. 35) Vol.-% | Propan und |
| 32 bis 72 Vol.-% | molekularer Stickstoff. |

Die Durchführung des zweiten Schrittes im Fall einer zweistufigen Partiäloxidation von Propylen zu Acrolein, nämlich die Partialoxidation vom Acrolein zur Acrylsäure, kann mit den beschriebenen Katalysatoren z. B. in einem Einzonen-Vielkontaktrohr-Festbettreaktor durchgeführt werden, wie es die DE-A 44 31 949 beschreibt. Dabei können Reaktiongsgasgemisch und Wärmeträger über den Reaktor betrachtet im Gleichstrom geführt werden. In der Regel wird das Produktgasgemisch der vorausgehenden erfindungsgemäßen Propylenpartialoxidation zu Acrolein grundsätzlich als solches (gegebenenfalls nach erfolgter Zwischenkühlung (diese kann indirekt oder direkt durch z. B. Sekundärluftzugabe erfolgen) desselben), d.h., ohne Nebenkomponentenabtrennung, in die zweite Reaktionsstufe, d.h. in die Acroleinpartialoxidation geführt.

Der für den zweiten Schritt, die Acroleinpartialoxidation, benötigte molekulare Sauerstoff kann (dabei) schon im Reaktionsgasausgangsgemisch für die erfindungsgemäße Propylenpartialoxidation zum Acrolein enthalten sein. Er kann aber auch teilweise oder vollständig dem Produktgasgemisch der ersten Reaktionsstufe, d. h., der erfindungsgemäßen Propylenpartialoxidation zum Acrolein, unmittelbar zugegeben werden (dies erfolgt vorzugsweise als (Sekundär)Luft, kann jedoch auch in Form von reinem Sauerstoff oder von Gemischen aus Inertgas oder Sauerstoff erfolgen). Unabhängig davon wie vorgegangen wird, weist das Beschickungsgasgemisch (das Reaktionsgasausgangsgemisch) einer solchen Partiafoxidation des Acroleins zu Acrylsäure, mit Vorteil nachfolgende Gehalte auf:

| | |
|---|---|
| 4 bis 8 Vol.-% | Acrolein, |
| 2,25 bzw. 4,5 bis 9 Vol.-% | molekularer Sauerstoff, |
| 6 bis 30 Vol.-% | Propan, |
| 32 bis 72 Vol.-% | molekularer Stickstoff und |
| 5 bis 15 Vol.-% | Wasserdampf. |

Bevorzugt weist das vorgenannte Reaktionsgasausgangsgemisch folgende Gehalte auf:

| | |
|---|---|
| 5 bis 8 Vol.-% | Acrolein, |
| 2,75 bzw. 5,5 bis 9 Vol.-% | molekularer Sauerstoff, |
| 10 bis 25 (bzw. 30)Vol.-% | Propan, |
| 40 bis 70 Vol.-% | molekularer Stickstoff und |
| 5 bis 15 Vol.-% | Wasserdampf. |

Ganz besonders bevorzugt weist das vorgenannte Reaktiongsgasausgangsgemisch folgende Gehalte auf:

| | |
|---|---|
| 5 bis 8 Vol.-% | Acrolein (bevorzugt 6 bis 7 Vol.-%), |
| 3 bzw. 6 bis 9 Vol.-% | molekularer Sauerstoff, |
| 10 bis 20 (bzw. 30) Vol-% | Propan (bevorzugt 10 bis 16 Vol.-%), |
| 50 bis 65 Vol.-% | molekularer Stickstoff und |
| 7 bis 13 Vol.-% | Wasserdampf, |

wobei die Vorzugsbereiche unabhängig voneinander gelten, mit Vorteil jedoch simultan realisiert werden.

Wie in der ersten Reaktionsstufe (Propylen → Acrolein) liegt auch in der zweiten Reaktionsstufe (Acrolein → Acrylsäure) der Reaktionsdruck üblicherweise im Bereich von 1 bis 3 bar, und die Gesamtraumbelastung des Katalysatorfestbetts mit Reaktionsgas(ausgangs)gemisch liegt vorzugsweise bei 1500 bis 4000 bzw. 6000 Nl/l•h oder mehr. Die Acroleinlast (die Acroleinbelastung des Katalysatorfestbetts) beträgt typisch 90 bis 190 Nl/l•h, oder bis 290 Nl/l•h oder mehr. Acroleinlasten oberhalb von 135 Nl/l•h, bzw. ≥ 140 Nl/l•h, oder ≥ 150 Nl/l•h, oder ≥ 160 Nl/l•h sind besonders bevorzugt, da das erfindungsgemäß enzusetzende Reaktionsgasausgangsgemisch infolge des Beiseins von Propan ebenfalls ein günstiges Heißpunktverhalten bedingt.

Der Acroleinumsatz, bezogen auf einmaligen Durchgang des Reaktionsgasgemischs durch das Katalysatorfestbett, beträgt zweckmäßig normalerweise ≥ 90 mol-% und die damit einhergehende Selektivität der Acrylsäurebildung ≥ 90 mol-%.

Bevorzugt wird der Einzonen-Vielkontaktrohr-Festbettreaktor mit dem Beschickungsgasgemisch ebenfalls von oben angeströmt. Als Wärmeaustauschmittel wird auch in der zweiten Stufe in zweckmäßiger Weise eine Salzschmelze, bevorzugt aus 60 Gew.-% Kaliumnitrat (KNO₃) und 40 Gew.-% Natriumnitrit (NaNO₂) oder aus 53 Gew.-% Kaliumnitrat (KNO₃), 40 Gew.-% Natriumnitrit (NaNO₂) und 7 Gew.-% Natriumnitrat (Na-NO₃) bestehend, eingesetzt. Über den Reaktor betrachtet können, wie bereits gesagt, Salzschmelze und Reaktionsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt zu beschicken:
- zunächst auf einer Länge von 50 bis 80 bzw. bis 70 % der Kontaktrohrlänge entweder nur Katalysator oder ein Gemisch aus Katalysator und Inertmaterial, wobei letzteres, bezogen auf die Mischung, einen Gewichtsanteil von bis zu 30 (bzw. 20) Gew.-% ausmacht (Abschnitt C);
- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 50 bzw. bis zu 40 Gew.-% ausmacht (Abschnitt B); und
- abschließend auf einer Länge von 5 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt. Wie ganz allgemein bei der heterogen katalysierten Gasphasenpartialoxidation von Acrolein zu Acrylsäure (insbesondere bei hohen Acroleinbelastungen des Katalysatorbetts und hohen Wasserdampfgehalten des Beschickungsgasgemischs), kann der Abschnitt B auch aus zwei aufeinanderfolgenden Katalysatorverdünnungen bestehen (zum Zweck der Minimierung von Heißpunkttempatur und Heißpunkttemperatursensitivität). Von unten nach oben zunächst mit bis zu 30 (bzw. 20) Gew.-% Inertmaterial und daran anschließend mit > 20 Gew.-% bis 50 bzw, bis 40 Gew.-% Inertmaterial, Der Abschnitt C ist dann bevorzugt unverdünnt.

Für eine Anströmung der Kontaktrohre von unten nach oben, wird die Kontaktrohrbeschickung in zweckmäßigerweise umgekehrt.

Vorgenannte Beschickungsvariante ist insbesondere dann zweckmäßig, wenn als Katalysatoren solche gemäß Herstellungsbeispiel 5 der DE-A 100 46 928 oder solche gemäß DE-A 198 15 281 und als Inertmaterial Ringe aus Steatit mit der Geometrie 7 mm x 7 mm x 4 mm oder 7 mm x 7 mm x 3 mm (jeweils Außendurchmesser x Höhe x Innendurchmessser) verwendet werden. Hinsichtlich der Salzbadtemperatur gilt das in der DE-A 443 19 49 Gesagte. Sie wird in der Regel so gewählt, daß der erzielte Acroleinumsatz bei einfachem Durchgang normalerweise ≥ 90 mol-%, bzw. ≥ 95 mol-% oder ≥ 99 mol-% beträgt.

Die Durchführung der Partialoxidation vom Acrolein zur Acrylsäure, kann mit den beschriebenen Katalysatoren aber auch z. B. in einem Zweizonen-Vielkontaktrohr-Festbettreaktor durchgeführt werden, wie es die DE-A 199 10 508 beschreibt. Für den Acroleinumsatz gilt das oben Genannte. Auch im Fall der Durchführung einer wie vorstehend beschriebenen Acroleinpartialoxidation als zweite Reaktionsstufe einer zweistufigen Propylenoxidation zu Acrylsäure in einem Zweizonen-Vielkontaktrohr-Festbettreaktor wird man zur Erzeugung des Beschickungsgasgemisches (Reaktionsgasausgangsgemischs) zweckmäßig unmittelbar das Produktgasgemisch der auf den ersten Schritt gerichteten Partialoxidation (gegebenenfalls nach erfolgter indirekter oder direkter (z. B. durch Zufuhr von Sekundärluft) Zwischenkühlung desselben) verwenden (wie sie vorstehend bereits beschrieben wurde). Der für die Acroleinpartialoxidation benötigte Sauerstoff wird vorzugsweise als Luft (gegebenenfalls aber auch als reiner molekularer Sauerstoff oder als Gemisch aus molekularem Sauerstoff und einem Inertgas) zugesetzt und z. B. dem Produktgasgemisch des ersten Schritts der zweistufigen Partialoxidation (Propylen → Acrolein) unmittelbar zugegeben. Er kann aber auch, wie bereits beschrieben, bereits im Reaktionsgasausgangsgemisch für die erste Reaktionsstufe enthalten sein.

Bei einer zweistufigen Partialoxidation von Propylen zu Acrylsäure mit unmittelbarer Weiterverwendung des Produktgasgemisches des ersten Schritts der Partialoxidation zur Beschickung des zweiten Schritts der Partialoxidation, wird man in der Regel zwei Einzonen-Vielkontaktrohr-Festbettreaktoren (bei hoher Reaktandenbelastung des Katalysatorbetts ist, wie ganz allgemein gültig, eine Gleichstromfahrweise zwischen Reaktionsgas und Salzbad (Wärmeträger) über den Rohrbündelreaktor betrachtet bevorzugt) oder zwei Zweizonen-Vielkontaktrohr-Festbettreaktoren hintereinanderschalten.

Eine gemischte Hintereinanderschaltung (Einzonen/Zweizonen oder umgekehrt) ist auch möglich.

Zwischen den Reaktoren kann sich ein Zwischenkühler (z. B. ein Rohrbündelwärmetauscher, durch dessen Rohre (die vorzugsweise zur Erhöhung des Wärmeübergangs metallische (vorzugsweise Edelstahl) Spiralen zentriert eingeführt enthalten) das Reaktionsgas geführt wird) befinden, der gegebenenfalls Inertschüttungen enthalten kann, die eine Filterfunktion ausüben können. Die Salzbadtemperatur von Vielkontaktrohrreaktoren für den ersten Schritt der zweistufigen Partialoxidation von Propylen zu Acrylsäure beträgt in der Regel 300 bis 400°C. Die Salzbadtemperatur von Vielkontaktrohrreaktoren für den zweiten Schritt der Partialoxidation von Propylen zu Acrylsäure, die Partialoxidation von Acrolein zu Acrylsäure, beträgt meist 200 bis 350°C. Außerdem werden die Wärmeaustauschmittel (bevorzugt Salzschmelzen) normalerweise in solchen Mengen durch die relevanten Vielkontaktrohr-Festbettreaktoren geführt, dass der Unterschied zwischen ihrer Eingangs- und ihrer Ausgangstemperatur in der Regel ≤ 5°C beträgt. Wie bereits erwähnt, können beide Schritte der Partialoxidation von Propylen zu Acrylsäure aber auch wie in der DE-A 101 21 592 beschrieben in einem Reaktor an einer Beschickung vollzogen werden.

Es sei auch nochmals erwähnt, dass ein Teil des Reaktionsgasausgangsgemischs für den ersten Schritt ("Propylen → Acrolein") aus der Partialoxidation kommendes Restgas sein kann.

Hierbei handelt es sich, wie bereits gesagt, um ein molekularen Sauerstoff enthaltendes Gas, das nach der Zielproduktabtrennung (Acrolein- und/oder Acrylsäureabtrennung) vom Produktgasgemisch der Partialoxidation verbleibt und teilweise als inertes Verdünnungsgas in die Beschickung für den ersten und/oder gegebenenfalls zweiten Schritt der Partialoxidation von Propylen zu Acrolein und/oder Acrylsäure rückgeführt werden kann.

Bevorzugt wird solches Propan, molekularen Sauerstoff und gegebenenfalls nicht umgesetztes Propylen enthaltendes Restgas erfindungsgemäß jedoch vorteilhaft ausschließlich als Kreisgas 1 in die heterogen katalysierte Propandehydrierung in der Reaktionszone A rückgeführt.

Insgesamt bildet ein Rohrbündelreaktor innerhalb dessen sich die Katalysatorbeschickurig längs der einzelnen Kontaktrohre mit Beendigung des ersten Reaktionsschrittes entsprechend ändert (derartige zweistufige Propylenpartialoxidationen im sogenannten "Single-Reactor" lehren z. B. die EP-A 91 13 13, die EP-A 97 98 13, die EP-A 99 06 36 und die DE-A 28 30 765) die einfachste Realisierungsform zweier Oxidationsstufen für die beiden Schritte der Partialoxidation von Propylen zu Acrylsäure. Gegebenenfalls wird dabei die Beschickung der Kontaktrohre mit Katalysator durch eine Inertmaterialschüttung unterbrochen.

Vorzugsweise werden die beiden Oxidationsstufen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet wird. Während die Kontaktrohre in der Regel von einem Wärmeträger umspült werden, erreicht dieser eine wie vorstehend beschrieben angebrachte Inertmaterialschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationszone verlässt, zu mindern. Die Reaktionstemperatur in der ersten Reaktionsstufe (Propylen → Acrolein) liegt in der Regel bei 300 bis 450°C, bevorzugt bei 320 bis 390°C. Die Reaktionstemperatur in der zweiten Reaktionsstufe (Acrolein, Acrylsäure) liegt in der Regel bei 200 bis 370°C, häufig bei 220 bis 330°C. Der Reaktionsdruck beträgt in beiden Oxidationszonen zweckmäßig 0,5 bis 5, vorteilhaft 1 bis 3 bar. Die Belastung (Nl/l•h) der Oxidationskatalysatoren mit Reaktionsgas beträgt in beiden Reaktionsstufen häufig 1500 bis 2500 Nl/l•h bzw. bis 4000 Nl/l•h. Die Belastung mit Propylen kann dabei bei 100 bis 200 oder 300 und mehr Nl/l•h liegen.

Prinzipiell können die beiden Oxidationsstufen beim erfindungsgemäßen Verfahren so gestaltet werden, wie es z. B. in der DE-A 198 37 517, der DE-A 199 10 506, der DE-A 199 10 508 sowie der DE-A 198 37 519 beschrieben ist.

In beiden Reaktionsstufen wirkt sich dabei ein Überschuss an molekularem Sauerstoff relativ zur reaktionsstöchiometrisch erforderlichen Menge vorteilhaft auf die Kinetik der jeweiligen Gasphasenpartialoxidation sowie auf die Katalysatorstandzeit aus. In der zweiten Reaktionsstufe ist er erfindungsgemäß zwingend.

Prinzipiell ist die erfindungsgemäß durchzuführende heterogen katalysierte Gasphasen-Partialoxidation von Propylen zu Acrylsäure aber auch in einem einzigen Einzonenrohrbündelreaktor wie folgt realisierbar. Beide Reaktionsschritte erfolgen in einem Oxidationsreaktor der mit einem oder mehreren Katalysatoren beschickt ist, deren Aktivmasse ein die Elemente Mo, Fe und Bi enthaltendes Multimetalloxid ist, das die Umsetzung beider Reaktionsschritte zu katalysieren vermag. Selbstredend kann sich auch diese Katalysatorbeschickung längs der Reaktionskoordinate kontinuierlich oder abrupt ändern. Natürlich kann bei einer Ausführungsform einer erfindungsgemäßen zweistufigen Partialoxidation von Propylen zu Acrylsäure in Gestalt zweier hintereinandergeschalteter Oxidationsstufen aus dem das die erste Oxidationsstufe verlassenden Produktgasgemisch in selbigem enthaltenes, in der ersten Oxidationsstufe als Nebenpro- , dukt entstandenes, Kohlenoxid und Wasserdampf bei Bedarf vor der Weiterleitung in die zweite Oxidationsstufe teilweise oder vollständig abgetrennt werden. Vorzugsweise wird man erfindungsgemäß eine Verfahrensweise wählen, die solch einer Abtrennung nicht vorsieht.

Als Quellen für eine zwischen beiden Oxidationsstufen durchgeführte Sauerstoffzwischeneinspeisung kommen, wie bereits gesagt, neben Luft (bevorzugt) sowohl reiner molekularer Sauerstoff als auch mit Inertgas wie CO₂, CO, Edelgasen, N₂ und/oder gesättigten Kohlenwasserstoffen verdünnter molekularer Sauerstoff in Betracht.

Durch Zudosieren von z. B. kalter Luft zum Produktgasgemisch der ersten Partialoxidaitonsstufe kann im Rahmen des erfindungsgemäßen Verfahrens auch auf direktem Weg eine Abkühlung desselben bewirkt werden, bevor es als Bestandteil eines Reaktionsgasausgangsgemisch für die zweite Partialoxidationsstufe weiterverwendet wird.

Erfindungsgemäß vorteilhaft erfolgt die Partialoxidation von Acrolein zu Acrylsäure wie in der EP-A 11 59 246, und ganz besonders bevorzugt wie in der WO 04/085365 sowie in der WO 04/085370 beschrieben. Erfindungsgemäß bevorzugt wird dabei als Acrolein enthaltendes Reaktionsgasausgangsgemisch jedoch ein Reaktionsgasausgangsgemisch verwendet, das das Produktgasgemisch einer erfindungsgemäßen Erststufenpartialoxidation von Propylen zu Acrolein ist, das gegebenenfalls mit soviel Sekundärluft ergänzt wurde, dass das Verhältnis von molekularem Sauerstoff zu Acrolein in dem resultierenden Reaktionsgasausgangsgemisch in jedem Fall 0,5 bis 1,5 beträgt. Die Schriften EP-A 1159246, WO 04/08536 und WO 04/085370 werden als integraler Bestandteil dieser Schrift betrachtet.

D.h., die erfindungsgemäße Partialoxidation von Acrolein zu Acrylsäure lässt sich vorteilhaft an einem Katalysatorfestbett mit erhöhter Acroleinbelastung durchführen, das wenigstens zwei Temperaturzonen aufweist.

Insgesamt wird man eine zweistufige Partialoxidation von Propylen zu Acrylsäure bevorzugt wie in der EP-A 1 159 248 bzw. in der WO 04/085367 oder WO 04/085369 beschrieben durchführen.

Der die erfindungsgemäß durchzuführende Partialoxidation (nach der ersten und/oder zweiten Reaktionsstufe) verlassende Produktgasgemischstrom B ist im Fall einer Herstellung von Acrolein und/oder Acrylsäure in der Regel im wesentlichen zusammengesetzt aus dem Zielprodukt Acrolein oder Acrylsäure oder dessen Gemisch mit Acrolein, nicht umgesetztem molekularem Sauerstoff (mit Blick auf die Lebensdauer der verwendeten Katalysatoren ist es vielfach günstig, wenn der Sauerstoffgehalt im Produktgasgemisch beider Partialoxidationsstufen z. B. noch wenigstens 1,5 bis 4 Vol.-% beträgt), Propan, nicht umgesetztem Propylen, molekularem Stickstoff, als Nebenprodukt entstandenem und/oder als Verdünnungsgas mitverwendetem Wasserdampf, als Nebenprodukt entstandenen und/oder als Verdünnungsgas mitverwendeten Kohlenoxiden, sowie geringen Mengen sonstiger niederer Aldehyde, niederer Alkancarbonsäuren (z. B. Essigsäure, Ameisensäure und Propionsäure) sowie Maleinsäureanhydrid, Benzaldehyd, aromatische Carbonsäuren und aromatische Carbonsäureanhydride (z.B. Phthalsäureanhydrid und Benzoesäure), gegebenenfalls weiteren Kohlenwasserstoffen, wie z. B. C4-Kohlenwasserstoffe (z. B. Buten-1 und eventuell sonstige Butene), und anderen inerten Verdünnungsgasen.

Als Verfahren zur Abtrennung von im Produktgasgemischstrom B enthaltenem Zielprodukt kommen für das erfindungsgemäße Verfahren prinzipiell alle im Stand der Technik diesbezüglich bekannten Verfahren in Betracht. Sie sind im wesentlichen dadurch gekennzeichnet, dass man das Zielprodukt z.B. durch absorptive und/oder kondensative Maßnahmen aus der gasförmigen in die kondensierte Phase überführt. Als Absorptionsmittel kommen dabei z. B. Wasser, wässrige Lösung und/oder organische Lösungsmittel in Betracht. Im Rahmen dieser "Kondensation" des Zielproduktes verbleibt normalerweise ein nicht in die kondensierte Phase übergehendes Restgas, das die vergleichsweise schwierig zu kondensierenden Bestandteile des Produktgasgemischstroms B umfasst. Dies sind üblicherweise vor allem diejenigen Komponenten, deren Siedepunkt bei Normaldruck (1 bar) ≤ -30°C beträgt (ihr Gesamtanteil am Restgas beträgt in der Regel ≥ 70 Vol.-%, häufig ≥ 80 Vol.-% und vielfach ≥ 90 Vol.-%). Dazu gehören in erster Linier nicht umgesetztes Propan, im Produktgasgemischstrom B verbliebener überschüssiger molekularer Sauerstoff sowie gegebenenfalls nicht umgesetztes Propylen. Zusätzlich wird das Restgas in der Regel inerte Verdünnungsgase wie z.B. N₂, CO₂, Edelgase (He, Ne, Ar etc.), CO sowie in geringem Umfang auch Acrylsäure, Acrolein und/oder H₂O (der Wasserdampfanteil am Restgas kann bis zu 25 Vol.-%, häufig bis zu 20 Vol.-%, oder bis zu 10 Vol.-%, vielfach aber auch unter 10 Vol.-% oder unter 5 Vol.-% betragen) sowie gegebenenfalls Nebenkomponenten wie Ethan, Methan und Ethylen enthalten. Dieses vorgenannte Restgas bildet (bezogen auf die darin enthaltene Menge an Propan) normalerweise die Hauptmenge (üblicherweise wenigstens 80%, bzw. wenigstens 90%, oder wenigstens 95% oder mehr) des in der Trennzone B gebildeten Restgases und wird daher in dieser Schrift u.a. auch als Hauptrestgas bezeichnet.

Erfindungsgemäß wird wenigstens eine nicht umgesetztes Propan, molekularen Sauerstoff, sowie gegebenenfalls nicht umgesetztes Propylen enthaltende Teilmenge dieses Restgases (Hauptrestgases) in Kreisgasfahrweise als gasförmiges Kreisgas 1 in die Reaktionszone A rückgeführt werden. Erfindungsgemäß zweckmäßig wird vielfach die Gesamtmenge dieses Restgases in die Reaktionszone A als Kreisgas 1 rückgeführt.

Insbesondere dann, wenn die Kondensation des Zielproduktes durch Absorption mittels eines organischen Lösungsmittels erfolgt, fällt in der Trennzone B in der Regel wenigstens ein zweites nicht umgesetztes Propan sowie gegebenenfalls nicht umgesetztes Propylen enthaltendes restliches Gas an (bezogen auf darin enthaltendes Propan ist seine Menge im Vergleich zur Hauptrestgasmenge normalerweise wesentlich geringer). Dies ist darauf zurückzuführen, dass die sich bildende kondensierte Phase in gewissem Umfang auch nicht umgesetztes Propan sowie gegebenenfalls nicht umgesetztes Propylen aufnimmt.

Im weiteren Verlauf der extraktiven, destillativen, kristallisativen und/oder desorptiven Abtrennung des Zielproduktes aus der kondensierten Phase, wird dieses nicht umgesetzte Propan sowie gegebenenfalls Propylen normalerweise als Bestandteil wenigstens einer weiteren Gasphase rückgewonnen und beim erfindungsgemäßen Verfahren vorzugsweise ebenfalls in die Reaktionszone A rückgeführt.

Dies kann z. B. im Gemisch mit dem Hauptrestgas erfolgen (in dieser Schrift dann Gesamtrestgas genannt). Es kann aber auch in Form eigenständiger in die Reaktionszone A rückzuführender Gasströme erfolgen. Diese eigenständigen rückzuführenden Gasströme können an Sauerstoff frei, oder auch Sauerstoff enthaltend (Nebenrestgas) sein (z. B. wenn er durch Strippen mittels Luft oder am Kopf einer mittels Luft als Polymerisationsinhibitor gespülten Rektifikationskolonne anfällt).

Sowohl Hauptrestgas, Gesamtrestgas als auch Nebenrestgas bilden im Sinne dieser Erfindung als Kreisgas 1 in die Reaktionszone A rückführbares nicht umgesetztes Propan, molekularen Sauerstoff und gegebenenfalls nicht umgesetztes Propylen enthaltendes Restgas. An molekularem Sauerstoff freies in der Trennzone B anfallendes nicht umgesetztes Propan sowie gegebenenfalls nicht umgesetztes Propylen enthaltendes restliches Gas kann erfindungsgemäß im Gemisch mit Hauptrestgas und/oder Nebenrestgas (d. h., z. B. als Bestandteil von Gesamtrestgas) z. B. als Bestandteil des Kreisgas 1 und/oder auch eigenständig (in diesem Fall handelt es sich nicht um in die Reaktionszone A im Sinne der Erfindung rückgeführtes Restgas) in die Reaktionszone A rückgeführt werden. Im letzteren Fall kann diese Rückführung ohne jedwede Beschränkung, d. h., z. B. auch als ein weiterer gasförmiger z. B. Kreisgas-3-strom erfolgen..lnsbesondere dann, wenn man beim erfindungsgemäßen Verfahren in einer ersten Trennzone A vom Produktgasgemisch im wesentlichen alle in ihm enthaltenen, von Propan und Propylen verschiedenen Bestandteile abtrennt und anschließend das dabei resultierende Produktgasgemisch A' zur Beschickung des wenigstens einen Oxidationsreaktors verwendet, wird man beim erfindungsgemäßen Verfahren im wesentlichen die Gesamtmenge der in der Trennzone B anfallenden, nicht umgesetztes Propan sowie gegebenenfalls nicht umgesetztes Propylen enthaltenden Gasströme in die Reaktionszone A rückführen, und dies vorzugsweise als Bestandteil von Gesamtrestgas als Kreisgas 1. Teilmengen könnten (wie z. B. in der DE-A 10 2004 032 129 beschrieben) gegebenenfalls aber auch für andere Zwecke, wie z. B. zur Energieerzeugung und/oder Synthesegasherstellung und/oder als Verdünnungsgas in der Reaktionszone B weiterverwendet werden. In der Regel wird man im vorbeschriebenen Fall jedoch wenigstens die Hälfte oder zwei Drittel (d. h., 50 Vol.-% oder 66,6 Vol.-%), bevorzugt wenigstens drei Viertel und ganz besonders bevorzugt die Gesamtmenge des vorgenannten in der Trennzone B anfallenden (jeweils individuell bezüglich des Hauptund/oder Neben- bzw. Gesamt-) Restgases in die Reaktionszone A, rückführen. Fällt in der Trennzone B nur ein nicht umgesetztes Propan, molekularen Sauerstoff und nicht umgesetztes Propylen enthaltender Restgasstrom an (dies ist häufig der Regelfall), wird dieser, insbesondere dann, wenn man beim erfindungsgemäßen Verfahren in einer ersten Trennzone A vom Produktgasgemisch im wesentlichen alle in ihm enthaltenen, von Propan und Propylen verschiedenen Bestandteile abtrennt und anschließend das dabei resultierende Produktgasgemisch A' zur Beschickung wenigstens eines Oxidationsreaktors verwendet, erfindungsgemäß bevorzugt vollständig (gegebenenfalls abzüglich einer in die Reaktionszone B als Verdünnungsgas geführten Teilmenge von identischer Zusammensetzung) als Kreisgas 1 in die Reaktionszone A rückgeführt. Er kann dann aber auch in zwei Teilmengen identischer Zusammensetzung aufgeteilt und, wie vorstehend beschrieben, nur eine Teilmenge als Kreisgas 1 in die Reaktionszone A rückgeführt und die andere Teilmenge anderweitig weiterverwendet werden. Fällt in der Trennzone B mehr als ein solcher Restgasstrom an, so können diese Restgasströme (wie bereits erwähnt) erfindungsgemäß gemeinsam (z. B. zusammengeführt) als Kreisgas 1 in die Reaktionszone A rückgeführt werden. Selbstverständlich kann die Rückführung dieser Restgasströme in die Reaktionszone A aber auch einzeln erfolgen. Auch kann eine Teilmenge oder die Gesamtmenge des Kreisgases 1 nicht in das der Reaktionszone A zugeführte Reaktionsgasgemischeingangsgas sondern erst längs des Reaktionspfads der heterogen katalysierten Dehydrierung des Propans in der Reaktionszone A in die Reaktionszone A rückgeführt werden. Unter dem Reaktionspfad der heterogen katalysierten Dehydrierung des Propans in der ersten Reaktionszone A soll dabei der Strömungsweg des im Reaktionsgasgemischeingangsstrom für die Reaktionszone A enthaltenen Propan durch die Reaktionszone A in Abhängigkeit vom dehydrierenden Umsatz (der Umsatz in der heterogen katalysierten Dehydrierung) dieses Propans verstanden.

Normalerweise besteht als Kreisgas 1 in die Reaktionszone A rückgeführtes Restgas beim erfindungsgemäßen Verfahren zu ≥ 70 Vol.-%, häufig zu ≥ 80 Vol.-% und vielfach zu ≥ 90 Vol.-%, meist zu ≥ 95 Vol.-%, oder zu ≥ 98 Vol.-% aus Bestandteilen, deren Siedepunkt bei Normaldruck (1 bar) ≤ -30°C beträgt.

Insbesondere dann, wenn man beim erfindungsgemäßen Verfahren in einer ersten Trennzone A vom Produktgasgemisch A im wesentlichen alle in ihm enthaltenen, von Propan und Propylen verschiedenen Bestandteile abtrennt und anschließend das dabei resultierende Produktgasgemisch A' zür Beschickung wenigstens eines Oxidationsreaktors verwendet, umfasst die Zusammensetzung des Kreisgases 1 in typischer Weise:
0 bis 2 Vol.-%, vielfach 0 bis 1 Vol.-%, häufig 0 bis 0,5 Vol.-% Propen;
0 bis 2 Vol.-%, vielfach 0 bis 1 Vol.-%, häufig 0 bis 0,5 Vol.-% Acrolein;
0 bis 0,5 Vol.-%, vielfach 0 bis 0,1 Vol.-%, häufig 0 bis 0,05 Vol.-% Acrylsäure;
0 bis 4 Vol.-%, vielfach 0 bis 2 Vol.-%, häufig 0 bis 1,5 Vol.-% COₓ;
10 bis 50 Vol.-%, vielfach 20 bis 30 Vol.-% Propan;
0 bis 70 Vol.%, vielfach 40 bis 70 Vol.-% N₂;
1 bis 10 Vol.-%, vielfach 2 bis 5 Vol.-%, häufig 2,5 bis 3,5 Vol.-% O₂ und
> 0 bis 15 Vol.-% H₂O.

Zusätzlich können in geringen Mengen Nebenkomponenten wie Ethan, Methan und Ethylen enthalten sein. Häufig weist das Kreisgas 1 beim erfindungsgemäßen Verfahren eine Temperatur von 50 bis 200°C, bzw. von 70 bis 130°C sowie einen Druck von 1,5 bis 5 bar, vorzugsweise von 3 bis 4 bar auf.

Gemäß dem vorgenannten kann das Zielprodukt aus dem Produktgasgemisch B also in an sich bekannter Weise in einer zweiten Trennzone B abgetrennt werden (z. B. durch partielle oder vollständige sowie gegebenenfalls fraktionierende Kondensation der Acrylsäure oder durch Absorption von Acrylsäure in Wasser oder in einem hochsiedenden hydrophoben organischen Lösungsmittel oder durch Absorption von Acrolein in Wasser oder in wässrigen Lösungen niederer Carbonsäuren sowie anschließende Aufarbeitung der Kondensate und/oder Absorbate; erfindungsgemäß bevorzugt wird das Produktgasgemisch B fraktionierend kondensiert werden; vgl. z. B. EP-A 1 388 533, EP-A 1 388 532, DE-A 102 35 847, EP-A 792 867, WO 98/01415, EP-A 1 095 411, EP-A 1 015 410, WO 99/50219, WO 00/53560, WO 02/09839, DE-A 102 35 847, WO 031041833, DE-A 102 23 058, DE-A 102 43 625, DE-A 103 36 386, EP-A 854 129, US-A 4,317,926, DE-A 198 37 520, DE-A 196 06 877, DE-A 190 50 1325, DE-A 102 47 240, DE-A 197 40 253, EP-A 695 736, EP-A 982 287, EP-A 1 041 062, EP-A 1 171 46, DE-A 43 08 087, DE-A 43 35 172, DE-A 44 36 243, DE-A 199 24 532, DE-A 103 32 758 sowie DE-A 199 24 533). Eine Acrylsäureabtrennung kann auch wie in der EP-A 982 287, der EP-A 982 289, der DE-A 103 36 386, der DE-A 101 15 277, der DE-A 196 06 877, der DE-A 197 40 252, der DE-A 196 27 847, der EP-A 920 408, der EP-A 1 068 174, der EP-A 1 066 239, der EP-A 1 066 240, der WO 00/53560, der WO 00153561, der DE-A 100 53 086 und der EP-A 982 288 vorgenommen werden. Vorzugsweise wird wie in Fig. 7 der WO/0196271 bzw. wie in der DE-A 10 2004 032 129 und deren äquivalenten Schutzrechten beschrieben abgetrennt. Günstige Abtrennweisen sind auch die in den Schriften WO 041063138, WO 04/35514, DE-A 102 43 625 und DE-A 102 35 847 beschriebenen Verfahren. Die Weiterverarbeitung einer dabei gewonnenen rohen Acrylsäure kann z. B. wie in den Schriften WO 01/77056; WO 03/041832, WO 02/055469, WO 03/078378 und WO 031041833 beschrieben erfolgen.

Gemeinsames Merkmal der vorgenannten Trennverfahren ist (wie bereits erwähnt), dass am Kopf der jeweiligen trennwirksame Einbauten enthaltenden Trennkolonne, in deren unteren Teil das Produktgasgemisch B, normalerweise nach vorheriger direkter und/oder indirekter Kühlung desselben, üblicherweise zugeführt wird, normalerweise ein Restgasstrom verbleibt, der im wesentlichen diejenigen Bestandteile des Produktgasgemischs B enthält, deren Siedepunkt bei Normaldruck (1 bar) ≤ -30°C beträgt (d. h., die schwer kondensierbaren oder auch leicht flüchtigen Bestandteile). Dazu zählt insbesondere im Produktgasgemisch B verbliebener überschüssiger molekularer Sauerstoff.

Im unteren Teil der Trennkolonne fallen normalerweise die schwerer flüchtigen Bestandteile des Produktgasgemischs B, einschließlich des jeweiligen Zielprodukts, in kondensierter Phase an. Die Restgasbestandteile sind in erster Linie Propan, gegebenenfalls in der Partialoxidation nicht umgesetztes Propylen, molekularer Sauerstoff sowie häufig die in der Partialoxidation sonstigen mitverwendeten inerten Verdünnungsgase, wie z. B. Stickstoff und Kohlendioxid. Wasserdampf kann je nach angewandtem Trennverfahren im Restgas nur noch in Spuren oder in Mengen von bis zu 20 Vol.-% oder mehr enthalten sein.
Von diesem Hauptrestgas wird erfindungsgemäß üblicherweise wenigstens eine (vorzugsweise Restgaszusammensetzung aufweisende) Propan, molekularen Sauerstoff und gegebenenfalls nicht umgesetztes Propylen enthaltende Teilmenge (vorzugsweise die Gesamtmenge, gegebenenfalls aber auch nur die Hälfte, oder zwei Drittel, oder drei Viertel dieser Gesamtmenge) als Kreisgas 1 in die Reaktionszone A rückgeführt. Restgasteilmengen können aber auch in eine oder in beide Stufen der Partialoxidation rückgeführt und/oder zum Zweck der Energieerzeugung verbrannt werden.

Natürlich können auch, wie in dieser Schrift sowie in der EP-A 117 146; US-A 3,161,670, DE-A 33 13 573 und DE-A 103 16 039 beschrieben, vorab einer Verwendung von Restgas als Kreisgas 1 aus dem Restgas insbesondere von Propan, Propylen und molekularem Sauerstoff verschiedene Bestandteile teilweise oder vollständig abgetrennt werden. Letzteres ist insbesondere dann zweckmäßig, wenn man beim erfindungsgemäßen Verfahren Produktgasgemisch A zur Beschickung des wenigstens einen Oxidationsreaktors mitverwendet.

Bei der Aufarbeitung der kondensierten Phase (zum Zweck der Abtrennung des Zielproduktes) können weitere restliche Gase anfallen, da normalerweise versucht werden wird, die insgesamt im Produktgasgemisch B enthaltene Menge an nicht umgesetztem . Propan in die Reaktionszone A zurückzuführen und im Rahmen der Zielproduktabtrennung wiederzugewinnen. Diese enthalten in der Regel zwar noch Propan sowie gegebenenfalls Propylen, häufig jedoch keinen molekularen Sauerstoff mehr. Üblicherweise werden sie mit dem Hauptrestgas zu einem Gesamtrestgas vereint als Kreisgas 1 in die Reaktionszone A rückgeführt. Es ist aber auch eine separate Verwertung bzw. Rückführung in die Reaktionszone A solcher weiteren restliche Gase möglich.

Durch die vorzugsweise vollständige Rückführung des Gesamtrestgases kann so im kontinuierlichen Betrieb eine kontinuierliche Umsetzung von Propan zu Acrylsäure und/oder Acrolein erfolgen.

Wesentlich ist dabei, dass durch die beschriebene Rückführung sowie durch die erfindungsgemäße Betriebsweise der Reaktionszone A in letzterer eine Überführung von Frischpropan zu Propylen mit nahezu hundertprozentiger Selektivität erreichbar ist.

Die Vorteilhaftigkeit einer solchen Verfahrensweise ist dabei sowohl bei niederen (≤ 30 mol-%) als auch bei hohen (≥ 30 mol-%) Dehydrierumsätzen (bezogen auf einmaligen Durchgang durch die Reaktionszone A) gegeben. Generell ist es erfindungsgemäß günstig, wenn der Wasserstoffgehalt im Reaktionsgasgemischeingangsstrom in die Reaktionszone A in einem wenigstens stöchiometrischen Verhältnis (bezüglich einer Sauerstoffverbrennung zu Wasser) zur in ihm enthaltenen Sauerstoffmenge steht.

An dieser Stelle sei nochmals festgehalten, dass die Abtrennung von Acrylsäure aus einem erfindungsgemäß erhaltenen Produktgasgemisch B bevorzugt so erfolgt, dass man das zuvor gegebenenfalls durch direkte und/oder indirekte Kühlung abgekühlte Produktgasgemisch B in einer trennwirksame Einbauten enthaltenden Kolonne unter Seitenabzug einer rohen Acrylsäure (z. B. in sich selbst) aufsteigend fraktionierend kondensiert und/oder mit Wasser bzw. wässriger Lösung absorbiert, wie es die WO 2004/035514 und die DE-A 102 43 625 beispielhaft beschreiben. Die entnommene rohe Acrylsäure wird nachfolgend bevorzugt einer Suspensionskristallisation unterworfen und das dabei gebildete Acrylsäuresuspensionskristallisat bevorzugt mittels einer Waschkolonne von verbliebener Mutterlauge abgetrennt. Mit Vorteil wird dabei als Waschflüssigkeit die Schmelze von in der Waschkolonne vorab abgetrennten Acrylsäurekristallen verwendet. Ferner ist die Waschkolonne bevorzugt eine solche mit erzwungenem Transport des Kristallbetts. Besonders bevorzugt handelt es sich um eine hydraulische oder um eine mechanische Waschkolonne. Im einzelnen kann der Beschreibung der WO 01/77056, der WO 03/041832 sowie der WO 03/041833 gefolgt werden. D. h., vorzugsweise wird verbliebene Mutterlauge in die fraktionierende Kondensation rückgeführt (vgl. auch EP-A 10 15 410). Der Nebenkomponentenauslass ist normalerweise unterhalb des Seitenabzugs der rohen Acrylsäure als purge-Strom.

Unter Anwendung von lediglich einer Kristallisationsstufe ist so Acrylsäure mit einer Reinheit ≥ 99,8 Gew.-% erhältlich, die sich in hervorragender Weise zur Herstellung von Superabsorbern auf Basis von Poly-Na-Acrylat eignet.

### Beispiele (konstruktives Material: Edelstahl vom Typ 1.4841)

### Beispiel 1

### I. Allgemeiner Versuchsaufbau der Reaktionszone A und deren Betriebsweise

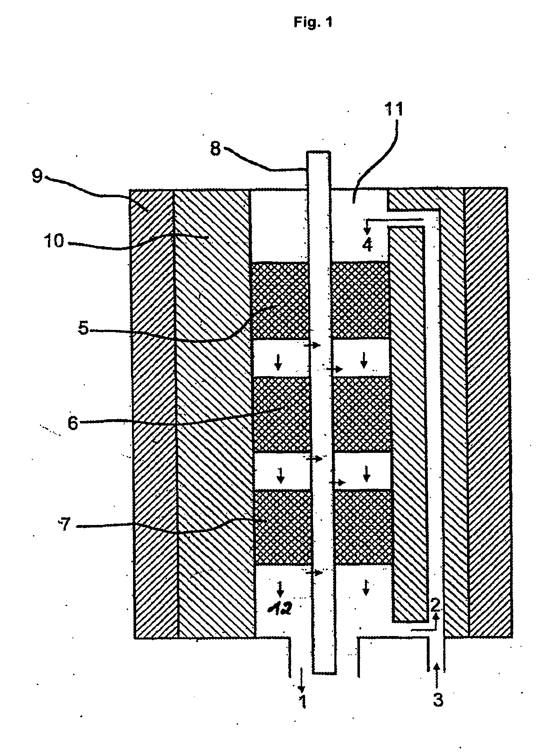

Die heterogen katalysierte partielle Propandehydrierung wird in einem Hordenschlaufenreaktor gemäß Figur 1 durchgeführt, auf die sich die numerischen Adressen im folgenden beziehen.

Ein vertikal stehender Rohrreaktor (11) (Innnendurchmesser: 80 mm) ist mit einer thermischen Isolierung (10) versehen in eine Stützheizung (9) eingehaust (ermöglicht weitgehende Adiabasie des Rohrreaktors). Die Temperatur der Stützheizung beträgt 500_{°}C. Mittig im Rohrreaktor befindet sich ein Zentralrohr (Außendurchmesser: 20 mm), welches eine Hülse für ein Zugthermoelement und eine Hülse für ein Stufenthermoelement enthält. Zusätzlich enthält es in den Rohrreaktor führende Leitungen, über die aus dem Rohrreaktor Reaktionsgasproben entnommen werden können, sowie in den Rohrreaktor führende Leitungen, über die in den Rohrreaktor Luft eingedüst werden kann.

Der Rohrreaktor enthält drei Horden (5, 6, 7), die aus drei identischen, auf einem Edelstahldrahtnetz aufgebrachten Schüttungen aus (in Strömungsrichtung in der genannten Abfolge angeordnet) Inertmaterial (Schütthöhe: 100 mm; Steatitkugeln des Durchmessers 1,5 bis 2,5 mm) und einer Mischung (Schütthöhe: 165 mm) aus Dehydrierkatalysator und Steatitkugeln (1,5 bis 2,5 mm Durchmesser) im Schüttvolumenverhältnis 1:1 bestehen. Die Gesamtschütthöhe beträgt damit jeweils 265 mm.

Der Dehydrierkatalysator ist eine Pt/Sn-Legierung, die mit den Elementen Cs, K und La in oxidischer Form promoviert ist und die auf die äußere und innere Oberfläche von ZrO₂. Si02 Mischoxidträgerstränglingen (mittlere Länge (gaußverteilt im Bereich von 3 mm bis 12 mm mit Maximum bei ca. 6 mm) : 6 mm, Durchmesser: 2 mm) in der Elementstöchiometrie (Massenverhältnisse einschließlich Träger) Pt_{0,3}Sn_{0,6}La_{3,0}Cs_{0,5}K_{0,2}(ZrO₂)_{88,3}(SiO₂)_{7,1} aufgebracht ist (Katalysatorvorläuferherstellung und Aktivierung zum aktiven Katalysator wie in Beispiel 4 der DE-A 10219879).

### Vor jeder Katalyastorhorde ist ein Mischelement angebracht.

Das aus der letzten Horde austretende Produktgasgemisch A (12) wird in zwei Hälften identischer Zusammensetzung aufgeteilt. Die eine Hälfte (2) wird als Produktgasgemisch A-Teilstrom 1 (Kreisgas 2) als Bestandteil des Reaktionsgasgemischeingangsstroms für die Reaktionszone A (4) in die Dehydrierung rückgeführt. Die andere Hälfte (1) wird als Produktgasgemisch A-Teilstrom 2 aus der Dehydrierzone (der Reaktionszone A) herausgeführt.

Der Reaktionsgasgemischeingangsstrom (4) besteht aus dem Produktgasgemisch A-Teilstrom-1 (auch Kreisgas 2 genannt) (2) sowie aus einem gasförmigen Ausgangsgemischstrom (3), der aus Wasserdampf, Kreisgas 1 aus der Partialoxidation, Frischpropan und molekularem Wasserstoff zusammengesetzt ist. Dieser Ausgangsgemischstrom (3) ist Treibstrahl einer Strahlpumpe (sie umfasst eine Treibdüse, eine Mischstrecke, einen Diffusor und einen Saugstutzen, wobei die Förderrichtung des durch die Treibdüse über die Mischstrecke und den Diffusor entspannten Treibstrahls in den Einlass der Reaktionszone A sowie die Saugwirkung des Saugstutzens in Richtung des den Produktgasgemischstrom A führenden Auslass der Reaktionszone A weist und dabei durch den im Saugstutzen erzeugten Unterdruck unter Aufteilung des Produktgasgemischstroms A in die beiden Teilströme 1 und 2 den Produktgasgemisch A-Teilstrom 1 ansaugt, bei gleichzeitiger Vermischung mit dem Treibstrahl durch die Mischstrecke über den Diffusor transportiert und den dabei gebildeten Reaktionsgasgemischeingangsstrom in den Einlass der Reaktionszone A entlässt), die den Produktgasgemischstrom A (12) wie beschrieben teilt und den Reaktionsgasgemischeingangsstrom für die Reaktionszone A (4) erzeugt.

Die Belastung der Katalysatorgesamtmenge (ohne Inertmaterial gerechnet) aller Horden mit Propan beträgt 350 NI/l•h.

Der Eingangsdruck des Reaktionsgasgemischeingangsstroms für die Reaktionszone A liegt bei 2,3 bar. Seine Temperatur beträgt 500°C. Der Druckverlust über den Dehydrierreaktor beträgt ca. 200 mbar. Vor (jeweils vor dem Mischelement) der zweiten und vor der dritten Katalysatorschüttung (in Strömungsrichtung) wird dem Reaktionsgasgemisch Luft (500°C, Reaktionsdruck) zugedüst. Die Menge ist so bemessen, dass die höchste Temperatur in der jeweils nachfolgenden Katalysatorschüttung 575 bis 580°C beträgt.

### II. Allgemeiner Versuchsaufbau der heterogen katalysierten zweistufigen Partialoxidation von Propylen zu Acrylsäure und deren Betriebsweise

### Versuchsanordnung

### Erste Reaktionsstufe:

Ein Reaktionsrohr (V2A Stahl; 30 mm Außendurchmesser, 2 mm Wandstärke, 28 mm Innendurchmesser, Länge: 350 cm, sowie ein in der Reaktionsrohrmitte zentriertes Thermorohr (10 mm Außendurchmesser) zur Aufnahme eines Thermoelements mit dem die Temperatur im Reaktionsrohr auf seiner gesamten Länge ermittelt werden kann) wird von oben nach unten wie folgt beschickt:
- Abschnitt 1:: 50 cm Länge
Steatitringe der Geometrie 7 mm x 7 mm x 4 mm (Außendurchmesser x Länge x Innendurchmesser) als Vorschüttung.
- Abschnitt 2:: 140 cm Länge
Katalysatorbeschickung mit einem homogenen Gemisch aus 20 Gew.-% (alternativ 30 Gew.-%) an Steatitringen der Geometrie 5 mm x 3 mm x 2 mm (Außendurchmesser x Länge x Innendurchmesser) und 80 Gew.-% (alternativ 70 Gew.-%) Vollkatalysator aus Abschnitt 3.
- Abschnitt 3:: 160 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2WO₃]_{0,5} [Mo₁₂Co_{5,5}Fe_{2,94}Si_{1,59}K_{0,08}Oₓ]₁). Alternativ kann hier auch einer der Katalysatoren BVK1 bis BVK11 aus Research Disclosure Nr. 497012 vom 29.08.2005 eingesetzt werden.

Von oben nach unten werden die ersten 175 cm mittels eines im Gegenstrom (zum Reaktionsgas) gepumpten Salzbades A thermostatisiert. Die zweiten 175 cm werden mittels eines im Gegenstrom (zum Reaktionsgas) gepumpten Salzbades B thermostatisiert.

### Zweite Reaktionsstufe:

Ein Reaktionsrohr (V2A Stahl; 30 mm Außendurchmesser, 2 mm Wandstärke, 28 mm Innendurchmesser, Länge: 350 cm, sowie ein in der Reaktionsrohrmitte zentriertes Thermorohr (10 mm Außendurchmesser) zur Aufnahme eines Thermoelements mit dem die Temperatur im Reaktionsrohr auf seiner gesamten Länge ermittelt werden kann) wird von oben nach unten wie folgt beschickt:
- Abschnitt 1:: 20 cm Länge
Steatitringe der Geometrie 7 mm x 7 mm x 4 mm (Außendurchmesser x Länge x Innendurchmesser) als Vorschüttung.
- Abschnitt 2:: 90 cm Länge
Katalysatorbeschickung mit einem homogenen Gemisch aus 25 Gew.-% (alternativ 30 Gew.-%) Steatit-Ringen der Geometrie 7 mm x 3 mm x 4 mm (Außendurchmesser x Länge x Innendurchmesser) und 75 Gew.-% (alternativ 70 Gew.-%) Schalenkatalysator aus Abschitt 4.
- Abschnitt 3:: 50 cm Länge
Katalysatorbeschickung mit einem homogenen Gemisch aus 15 Gew.-% (alternativ 20 Gew.-%) an Steatit-Ringen der Geometrie 7 mm x 3 mm x 4 mm (Außendurchmesser x Länge x Innendurchmesser) und 85 Gew.-% (alternativ 80 Gew.-%) Schalenkatalysator aus Abschnitt 4.
- Abschnitt 4:: 190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: Mo₁₂V₃W_{1,2}Cu_{2,4}Oₓ).

Von oben nach unten werden die ersten 175 cm mittels eines im Gegenstrom (zum Reaktionsgas) gepumpten Salzbades C thermostatisiert. Die zweiten 175 cm werden mittels eines im Gegenstrom gepumpten Salzbades D thermostatisiert.

### III. Verfahren zur Herstellung von Acrylsäure aus Propan (beschrieben wird der stationäre Betriebszustand)

Dem ersten Katalysatorbett eines wie in I beschriebenen Hordenreaktor wird ein Reaktionsgasgemischeingangsstrom zugeführt, der folgende Gehalte aufweist (Vol.-% bezogen auf Gesamtgas):

| | Vol.-% |
|---|---|
| Acrylsäure | 0,01 |
| Essigsäure | 0,015 |
| Wasser | 9,23 |
| 1-Buten | 0,01 |
| iso-Buten | 0,02 |
| Propan | 18,46 |
| Propylen | 3,98 |
| Ethan | 1,16 |
| Ethylen | 0,22 |
| CO₂ | 2,34 |
| CO | 0,26 |
| N₂ | 59,7 |
| O₂ | 1,62 |
| CH₄ | 0,12 |
| H₂ | 2,83 |

Er wird erzeugt (er enthält) aus (in der Reihenfolge Kreisgas 1 (23°C, 3,1 bar), Frischpropan, (25°C, 4 bar), Wasserdampf (200°C, 2,5 bar), Wasserstoff (25°C, 8 bar), Dehydrierkreisgas (Kreisgas 2) (600°C, 1,9 bar)):
- 41,9 Vor.-% Restgas aus der Partialoxidation (Kreisgas 1) das folgende Gehalte aufweist:

| | Vol.-% |
|---|---|
| Acrylsäure | 0,02 |
| Essigsäure | 0,04 |
| H₂O | 2,73 |
| iso-Buten | 0,01 |
| Acrolein | 0,05 |
| Propan | 17,30 |
| Propylen | 0,32 |
| Ethan | 1,20 |
| Ethylen | 0,22 |
| CO₂ | 2,41 |
| CO | 0,61 |
| N₂ | 71,21 |
| O₂ | 3,87 |

- 3,9 Vol.-% Frischpropan, das folgende Gehalte aufweist:

| | Vol.-% |
|---|---|
| Propan | 98,91 |
| iso-Butan | 0,05 |
| Propylen | 0,1 |
| Ethan | 0,92 |
| Ethylen | 0,01 |

- 1,02 Vol.-% molekularem Wasserstoff
- 2,03 Vol.-% Wasserdampf und
- 51,15 Vol.-% Dehydrierkreisgas (Produktgasgemisch A-Teilstrom 1 bzw. Kreisgas 2)

Restgas (Kreisgas 1), Frischpropan, Wasserdampf und Wasserstoff werden dabei in der genannten Reihenfolge zum Treibstrahlgemischstrom vereinigt und durch indirekten Wärmeaustausch mit dem Produktgasgemisch A-Teilstrom 2 auf 500°C, 2,3 bar gebracht.

Der resultierende Produktgasgemisch A-Teilstrom 2 weist folgende Gehalte auf:

| | Vol.-% |
|---|---|
| H₂O | 11,84 |
| iso-Buten | 0,01 |
| Propan | 14,32 |
| Propylen | 7,52 |
| Ethan | 1,21 |
| Ethylen | 0,26 |
| CO₂ | 2,61 |
| N₂ | 58,41 |
| O₂ | 0,23 |
| H₂ | 3,55 |

Das im Produktgasgemisch A-Teilstrom 2 enthaltene Propan und Propylen wird durch Absorption in technischem Tetradecan der Fa. Haltermann, DE vom Typ PKWF 4/7 af als Absorptionsmittel (alternativ kann hier auch LINPAR 14-17 der Firma CONDEA Augusta S.p.A (Italien) eingesetzt werden) absorptiv abgetrennt und mittels Luft unter Erhalt des folgenden Beschickungsgases für die Partialoxidation wieder freigestrippt (dabei wird wie in der DE-A 10 2004 032 129 beschrieben verfahren), das die folgenden Gehalte aufweist:

| | Vol.-% |
|---|---|
| H₂O | 2,39 |
| Tetradekan | 0,01 |
| iso-Buten | 0,01 |
| Propan | 15,15 |
| Propylen | 7,95 |
| Ethan | 1,10 |
| Ethylen | 0,20 |
| CO₂ | 1,05 |
| N₂ | 58,99 |
| O₂ | 15,16 |

Mit diesem Beschickungsgasgemisch (es liegt außerhalb des Explosionsbereichs) wird die beschriebene erste Partialoxidationsreaktionsstufe beschickt. Die Propylenbelastung der Festbettkatalysatorbeschickung wird zu 185 Nl/l•h gewählt. Der Druck am Eingang der ersten Reaktionsstufe beträgt 3,1 bar. T_{A} = 322°C; T_{B} = 328°C.

Das die erste Reaktionsstufe verlassende Produktgasgemisch weist folgende Gehalte auf:

| | Vol.% |
|---|---|
| Acrylsäure | 0,46 |
| Essigsäure | 0,14 |
| H₂O | 10,65 |
| 1-Buten | 0,01 |
| Acrolein | 6,99 |
| Propan | 15,16 |
| Propylen | 0,17 |
| Ethan | 1,10 |
| Ethylen | 0,20 |
| CO₂ | 1,62 |
| CO | 0,23 |
| N₂ | 57,02 |
| O₂ | 6,25 |

U^{P}_{A}, der Propenumsatz am Ende der Reaktionszone A, beträgt 64,5 mol.-%.
U^{P}_{B}, der Propenumsatz am Ende der Reaktionszone B, beträgt 94,9 mol.-%.

Dem Produktgasgemisch der ersten Stufe wird soviel Luft (25°C) zudosiert, dass das molare Verhältnis O₂: Acrolein im resultierenden Gemisch 1,25 beträgt.

Mit diesem Gemisch wird dann die zweite Reaktionsstufe unmittelbar beschickt (T = 231,7°C).
Die Acroleinbelastung des Katalysatorfestbetts beträgt 152 NI/l• h.
Tc = 263°C; T_{D} = 269°C. Der Druck am Eingang der zweiten Reaktionsstufe beträgt 2,1 bar.

Das die zweite Reaktionsstufe verlassende Produktgasgemisch B weist folgende Gehalte auf:

| | Vol.-% |
|---|---|
| Acrylsäure | 6,72 |
| Essigsäure | 0,22 |
| H₂O | 11,06 |
| Formaldehyd | 0,14 |
| Acrolein | 0,05 |
| Ameisensäure | 0,03 |
| Maleinsäureanhydrid | 0,06 |
| Benzolsäure | 0,01 |
| Propan | 14,62 |
| Propylen | 0,28 |
| Ethan | 1,02 |
| Ethylen | 0,18 |
| CO₂ | 2,03. |
| CO | 0,52 |
| N₂ | 59,86 |
| O₂ | 3,20 |
| Propionsäure | 0,0032 |

U^{A}_{C}, der Acroleinumsatz am Ende der Reaktionszone C, beträgt 68,1 mol.-%.

U^{A}_{D}, der Acroleinumsatz am Ende der Reaktionszone D, beträgt 99,3 mol.-%.

In beiden Reaktionsstufen durchströmt das Reaktionsgasgemisch die beiden Kontaktrohre von oben nach unten.

Die Gehaltsanalysen erfolgen mittels gaschromatographischer Analyse.

Die Acrylsäure wird aus dem Produktgasgemisch wie in den beispielhaften Ausführungen der DE-A 10 2004 032 129 abgetrennt und das molekularen Sauerstoff enthaltende Restgas als Kreisgas 1 in die heterogen katalysierte Dehydrierung rückgeführt.

Das Verfahren kann auch wie beschrieben, jedoch mit dem Unterschied durchgeführt werden, dass in der Reaktionszone A jede Katalysatorhorde nur mit der gleichen Menge Dehydrierkatalysator beschickt ist, d. h., ohne Mitverwendung von Inertmaterial zu Verdünnungszwecken.

Der beschriebene stationäre Betriebszustand wird für die Zeitdauer von 2 Minuten dadurch gestört, dass man die dem Produktgasgemisch der ersten Partialoxidationsstufe zudosierte Menge an Luft soweit erhöht, dass der Gehalt an O₂ im Produktgasgemisch B zunächst auf 3,40 Vol.-% ansteigt.

Der Restsauerstoffgehaft von 3,20 Vol.-% im Produktgasgemisch B lässt sich während der 2-minütigen Betriebsstörung dadurch wiedergewinnen und nachfolgend aufrechterhalten, dass man die Frischpropanmenge zum Reaktionsgasgemischeingangsstrom für die Reaktionszone A unter ansonsten unveränderten Bedingungen um 1/100 bis 3/100 (bezogen auf den Vorwert) erhöht. Die Reaktionstemperatur in der Reaktionszone A bleibt im wesentlichen erhalten (unverändert).

Unter Anwendung des erfindungsgemäßen Regelprinzips ist ein dauerhafter stationärer Betrieb möglich, der stets nur geringfügig von der Ideallinie abweicht.

### Beispiel 2

### I. Allgemeiner Versuchsaufbau der Reaktionszone A und deren Betriebsweise

Die heterogen katalysierte partielle Propandehydrierung wird in einem nachfolgend beschriebenen Hordenreaktor im geraden Durchgang (d.h., ohne Schlaufenfahrweise) durchgeführt. Ein vertikal stehender Rohrreaktor (Innendurchmesser: 80 mm) ist mit einer thermischen Isolierung versehen in eine Stützheizung eingehaust (ermöglicht weitgehende Adiabasie des Rohrreaktors). Die Temperatur der Stützheizung beträgt 500°C. Mittig im Rohrreaktor befindet sich ein Zentralrohr (Außendurchmesser: 20 mm), welches eine Hülse für ein Zugthermoelement und eine Hülse für ein Stufenthermoelement enthält. Zusätzlich enthält es in den Rohrreaktor führende Leitungen, über die aus dem Rohrreaktor Reaktionsgasproben entnommen werden können, sowie in den Rohrreaktor führende Leitungen, über die in den Rohrreaktor Luft eingedüst werden kann.

Der Rohrreaktor enthält drei hintereinander angeordnete Horden, die aus drei identischen, auf einem Edelstahldrahtnetz aufgebrachten Schüttungen aus (in Strömungsrichtung in der genannten Abfolge angeordnet) aus Inertmaterial (Schütthöhe: 26 mm; Steatitkugeln des Durchmessers 1,5 bis 2,5 mm) und einer Mischung (Schütthöhe: 174 mm) aus Dehydrierkatalysator und Steatitkugeln (1,5 bis 2,5 mm Durchmesser) im Schüttvolumenverhältnis 1:3 bestehen. Die Gesamtschütthöhe beträgt damit jeweils 200 mm. Als Dehydrierkatalysator wird der Dehydrierkatalysator aus Beispiel 1 verwendet. Vor jeder Katalysatorhorde ist ein Mischelement angebracht.

Die Belastung der Katalysatorgesamtmenge (ohne Inertmaterial gerechnet) aller Horden mit Propan beträgt 1500 NI/l•h.

Der Eingangsdruck des Reaktionsgasgemischeingangsstroms für die Reaktionszone A liegt bei 3,1 bar. Seine Temperatur beträgt 450°C. Der Druckverlust über den Dehydrierreaktor beträgt ca. 400 mbar. Vor (jeweils vor dem Mischelement) der zweiten und vor der Dritten Katalysatorschüttung (in Strömungsrichtung) wird dem Reaktionsgasgemisch Luft (500°C, Reaktionsdruck) zugedüst. Die Menge ist so bemessen, dass die höchste Temperatur in der jeweils nachfolgenden Katalysatorschüttung 575 bis 580°C beträgt.

### II. Allgemeiner Versuchsaufbau der heterogen katalysierten zweistufigen Partialoxidation von Propylen zu Acrylsäure und deren Betriebsweise

Wie in Beispiel 1.

### III. Verfahren zur Herstellung von Acrylsäure aus Propan (beschrieben wird der stationäre Betriebszustand)

Dem ersten Katalysatorbett des wie in 1. beschriebenen Hordenreaktors wird ein Reaktionsgasgemischeingangsstrom zugeführt, der folgende Gehalte aufweist: (Vol.% bezogen auf Gesamtgas):

| | Vol.-% |
|---|---|
| Acrylsäure | 0,033 |
| Essigsäure | 0,017 |
| Wasser | 9,23 |
| n-Butan | 0,0004 |
| iso-Butan | 0,008 |
| Ameisensäure | 0,0005 |
| Acrolein | 0,024 |
| Propionsäure | 0,0001 |
| Methacrylsäure | 0,0001 |
| Furfural | 0,0001 |
| Propan | 32,93 |
| Propylen | 0,19 |
| Ethylen | 0,035 |
| Ethan | 0,18 |
| N₂ | 51,33 |
| O₂ | 2,97 |
| CO₂ | 1,7 |
| H₂ | 0,09 |
| CO | 0,38 |

Es wird erzeugt (er enthält) aus (in der Reihenfolge Kreisgas 1 (23°C, 3,1 bar), Frischpropan (25°C, 4 bar), Wasserdampf (200°C, 2,5 bar)):
- 86,61 Vol.-% Restgas aus der Partialoxidation (Kreisgas 1) das folgende Gehalte aufweist:

| | Vol.-% |
|---|---|
| Acrylsäure | 0,038 |
| Essigsäure | 0,020 |
| H₂O | 2,99 |
| n-Butan | 0,0004 |
| iso-Butan | 0,0052 |
| Ameisensäure | 0,0006 |
| Acrolein | 0,028 |
| Propionsäure | 0,0001 |
| Furfural | 0,0001 |
| Propan | 30,4 |
| Propylen | 0,21 |
| Ethylen | 0,037 |
| Ethan | 0,14 |
| N₂ | 59,32 |
| O₂ | 3,43 |
| CO₂ | 1,98 |
| H₂ | 0,1 |
| CO | 0,44 |

- 6,75 Vol.-% Frischpropan, das folgende Gehalte aufweist:

| | Vol.-% |
|---|---|
| n-Butan | 0,0007 |
| iso-Butan | 0,045 |
| Propan | 98 |
| Propylen | 0,11 |
| Ethylen | 0,014 |
| Ethan | 0,92 |

und
- 6,64 Vol.-% Wasserdampf.

Restgas (Kreisgas 1), Frischpropan und Wasserdampf werden dabei in der genannten Reihenfolge vereinigt und durch indirekten Wärmeaustausch mit dem Produktgasgemisch A oder mittels elektrischer Beheizung auf 450°C, 3,1 bar gebracht.

Das resultierende Produktgasgemisch A weist folgende Gehalte auf (Angaben in Vol.-%):

| | Vol.-% |
|---|---|
| H₂O | 0,845 |
| n-Butan | 0,0003 |
| iso-Butan | 0,0051 |
| 1-Buten | 0,0001 |
| iso-Buten | 0,0022 |
| Propan | 29,07 |
| Propylen | 6,84 |
| Ethylen | 0,037 |
| Ethan | 0,13 |
| N₂ | 48,72 |
| O₂ | 12,43 |
| CO₂ | 0,806 |
| H₂ | 0,1 |

Das im Produktgasgemisch A enthaltene Propan und Propylen wird durch Absorption in technischem Tetradecan der Fa. Haltermann, DE vom Typ PKWF 4/7 af als Absorptionsmittel (alternativ kann hier auch LINPAR 14-17 der Firma CONDEA Augusta S.p.A. (Italien) eingesetzt werden) absorptiv abgetrennt und mittels Luft unter Erhalt des folgenden Beschickungsgases für die Partialoxidation wieder freigestrippt (dabei wird wie in der DE-A 10 2004 032 129 beschrieben verfahren), das die folgenden Gehalte aufweist (Angaben in Vol.-%):

| | Vol.-% |
|---|---|
| H₂O | 2,5 |
| Tetradekan | 0,006 |
| n-Butan | 0,0003 |
| iso-Butan | 0,0051 |
| 1-Buten | 0,0001 |
| iso-Buten | 0,0022 |
| Propan | 28,57 |
| Propylen | 6,73 |
| Ethylen | 0,037 |
| Ethan | 0,13 |
| N₂ | 47,9 |
| O₂ | 12,22 |
| CO₂ | 0,88 |
| H₂ | 0,097 |

Mit diesem Beschickungsgasgemisch (es liegt außerhalb des Explosionsbereichs) wird die beschriebene erste Partialoxidationsstufe beschickt. Die Propylenbelastung der Festbettkatalysatorbeschickung wird zu 145 Nl/l•h gewählt. Der Druck am Eingang der ersten Reaktionsstufe beträgt 3,1 bar. T_{A} = 324°C; T_{B} = 328°C.

Das die erste Reaktionsstufe verlassende Produktgasgemisch weist folgende Gehalte auf (Angaben in Vol.-%):

| | Vol.-% |
|---|---|
| Acrylsäure | 0,33 |
| Essigsäure | 0,026 |
| Dampf | 9,28 |
| n-Butan | 0,0003 |
| iso-Butan | 0,0051 |
| Acrolein | 5,90 |
| Propionsäure | 0 |
| Methacrylsäure | 0,0002 |
| Propan | 28,52 |
| Propylen | 0,38 |
| Ethylen | 0,037 |
| Ethan | 0,13 |
| N₂ | 47,89 |
| O₂ | 5,1 |
| CO₂ | 1,3 |
| CO | 0,18 |

U^{P}_{A}, der Propenumsatz am Ende der Reaktionszone A, beträgt 64,5 mol.-%.

U^{P}_{B}, der Propenumsatz am Ende der Reaktionszone B, beträgt 94,9 mol.-%.

Dem Produktgasgemisch der ersten Stufe wird soviel Luft (25°C) zudosiert, dass das molare Verhältnis O₂: Acrolein im reslutierenden Gemisch 1,22 beträgt.

Mit diesem Gemisch wird dann die zweite Reaktionsstufe unmittelbar beschickt (T = 231,7°C).

Die Acroleinbelastung des Katalysatorfestbetts beträgt 121 Nl/l•h.
T_{C} = 265°C; T_{D} = 269°C. Der Druck am Eingang der zweiten Reaktionsstufe beträgt 2,1 bar.

Das die zweite Reaktionsstufe verlassende Produktgasgemisch B weist folgende Gehalte auf (Angaben in Vol.-%):

| | Vol.-% |
|---|---|
| Acrylsäure | 5,52 |
| Essigsäure | 0,11 |
| H₂O · | 9,44 |
| n-Butan | 0,0003 |
| iso-Butan | 0,0047 |
| Ameisensäure | 0,0063 |
| Formaldehyd | 0,044 |
| Acrolein | 0,025 |
| Propionsäure | 0,0029 |
| Methacrylsäure | 0,0002 |
| Furfural | 0,0017 |
| Benzaldehyd | 0,0008 |
| Maleinsäureanhydrid | 0,043 |
| Propan - | 26,47 |
| Propylen | 0,19 |
| Ethylen | 0,034 |
| Ethan | 0,12 |
| N₂ | 51,89 |
| O₂ | 3 |
| CO₂ | 1,6 |
| H₂ | 0,09 |
| CO | 0,39 |

U^{A}_{C}, der Acroleinumsatz am Ende der Reaktionszone C, beträgt 68,1 mol.-%.

U^{A}_{D}, der Acroleinumsatz am Ende der Reaktionszone D, beträgt 99,2 mol.-%.

In beiden Reaktionsstufen durchströmt das Reaktionsgasgemisch die beiden Kontaktrohre von oben nach unten.

Die Gehaltsanalysen erfolgen mittels gaschromatographischer Analyse.

Die Acrylsäure wird aus dem Produktgasgemisch wie in den beispielhaften Ausführungen der DE-A 10 2004 032 129 abgetrennt und das molekulare Sauerstoff enthaltende Restgas als Kreisgas 1 in die heterogen katalysierte Dehydrierung rückgeführt.

Das Verfahren kann auch wie beschrieben, jedoch mit dem Unterschied durchgeführt werden, dass in der Reaktionszone A jede Katalysatorhorde nur mit der gleichen Menge Dehydrierkatalysator beschickt ist, d.h., ohne Mitverwendung von Inertmaterial zu Verdünnungszwecken.

Der beschriebene stationäre Betriebszustand wird für die Zeitdauer von 2 Minuten dadurch gestört, dass man die dem Produktgasgemisch der ersten Partialoxidationsstufe zudosierte Menge an Luft soweit vermindert, dass der Gehalt an O₂ im Produktgasgemisch B zunächst auf 2,80 Vol.-% fällt.

Der Restsauerstoffgehalt von 3,20 Vol.-% im Produktgasgemisch B lässt sich während der 2-minütigen Betriebsstörung dadurch wiedergewinnen und nachfolgend aufrechterhalten, dass man die Frischpropanmenge zum Reaktionsgasgemischeingangsstrom für die Reaktionszone A unter ansonsten unveränderten Bedingungen um 1/100 bis 3/100 (bezogen auf den Vorwert) erniedrigt. Die Reaktionstemperatur in der Reaktionszone A bleibt im wesentlichen erhalten (unverändert).

Unter Anwendung des erfindungsgemäßen Regelprinzips ist ein dauerhafter stationärer Betrieb möglich, der stets nur geringfügig von der Ideallinie abweicht.

US Provisional Patent Application No. 60/732,658, eingereicht am 03.11.2005, ist in die vorliegende Patentanmeldung durch Literaturhinweis eingefügt.

Im Hinblick auf die obengenannten Lehren sind zahlreiche Änderungen und Abweichungen von der vorliegenden Erfindung möglich.

Man kann deshalb davon ausgehen, dass die Erfindung, im Rahmen der beigefügten Ansprüche, anders als hierin spezifisch beschrieben, ausgeführt werden kann.

## Patentansprüche

1. Verfahren zum stabilen Betreiben eines kontinuierlich ausgeübten Herstellprozesses zur Erzeugung von Acrolein, oder Acrylsäure oder deren Gemisch aus Propan, bei dem man
A) in einer ersten Reaktionszone A Propan im Beisein von molekularem Sauerstoff einer heterogen katalysierten Dehydrierung unterwirft, wobei ein Propan und Propylen enthaltendes Produktgasgemisch A erhalten wird,
B) gegebenenfalls Produktgasgemisch A in eine erste Trennzone A führt, um in selbiger von im Produktgasgemisch A enthaltenen, von Propan und Propylen verschiedenen, Bestandteilen eine Teilmenge oder mehr abzutrennen und dadurch ein verbleibendes, Propan und Propylen enthaltendes, Produktgasgemisch A' zu erzeugen,
C) Produktgasgemisch A oder Produktgasgemisch A' in einer zweiten Reaktionszone B zur Beschickung wenigstens eines Oxidationsreaktors verwendet und in dem wenigstens einen Oxidationsreaktor im Produktgasgemisch A oder im Produktgasgemisch A' enthaltenes Propylen einer selektiven heterogen katalysierten partiellen Gasphasenoxidation mit molekularem Sauerstoff zu einem Acrolein, oder Acrylsäure, oder deren Gemisch als Zielprodukt, nicht umgesetztes Propan, überschüssigen molekularen Sauerstoff und gegebenenfalls nicht umgesetztes Propylen enthaltenden Produktgasgemisch B unterwirft,
D) in einer zweiten Trennzone B im Produktgasgemisch B enthaltenes Zielprodukt abtrennt und von dem dabei verbleibenden, Propan, molekularen Sauerstoff sowie gegebenenfalls nicht umgesetztes Propylen enthaltenden Restgas wenigstens eine nicht umgesetztes Propan, molekularen Sauerstoff und gegebenenfalls nicht umgesetztes Propylen enthaltende Teilmenge als molekularen Sauerstoff enthaltendes Kreisgas 1 in Kreisgasfahrweise in die Reaktionszone A rückführt,
E) dem kontinuierlich ausgeübten Herstellprozess über wenigstens eine der Zonen aus der Gruppe umfassend die Reaktionszone A, die Trennzone A, die Reaktionszone B und die Trennzone B Frischpropan mit einer Zufuhrrate zuführt, die im stabilen Betriebszustand des Herstellprozesses einen vorgegebenen stationären Wert aufweist, und
F) den Gehalt des Produktgasgemischs B an molekularem Sauerstoff kontinuierlich ermittelt und mit dem für den stabilen Betriebszustand des Herstellprozesses vorgegebenen stationären Zielgehalt an molekularem Sauerstoff im Produktgasgemisch B vergleicht,
**dadurch gekennzeichnet, dass** man
- für den Fall, dass der zu einem Zeitpunkt ermittelte Gehalt an molekularem Sauerstoff im Produktgasgemisch B größer als der stationäre Zielgehalt ist, im Anschluss an diesen Zeitpunkt dem Herstellprozess Frischpropan mit einer Zufuhrrate zuführt, die größer als ihr stationärer Wert ist, und
- für den Fall, dass der zu einem Zeitpunkt ermittelte Gehalt an molekularem Sauerstoff im Produktgasgemisch B kleiner als der stationäre Zielgehalt ist, im Anschluss an diesen Zeitpunkt dem Herstellprozess Frischpropan mit einer Zufuhrrate zuführt, die kleiner als ihr stationärer Wert ist.

2. Verfahren nach Anspruch 1, **dadurch gekennzeichnet, dass** die kontinuierliche Ermittlung des Gehalts an molekularem Sauerstoff des Produktgasgemischs B darauf beruht, dass ein Signal gewonnen wird, das mit dem Sauerstoffgehalt des Produktgasgemischs B korreliert ist.

3. Verfahren nach Anspruch 2, **dadurch gekennzeichnet, dass** das Signal dadurch gewonnen wird, dass elektromagnetische Strahlung mit einer Wellenlänge, bei der molekularer Sauerstoff elektromagnetische Strahlung absorbiert, durch Produktgasgemisch B geleitet und der nichtabsorbierte Anteil der elektromagnetischen Strahlung gemessen wird.

4. Verfahren nach Anspruch 3, **dadurch gekennzeichnet, dass** die Wellenlänge 759,5 nm bis 768 nm beträgt.

5. Verfahren nach Anspruch 2, **dadurch gekennzeichnet, dass** die Ermittlung des Gehalts an molekularem Sauerstoff des Produktgasgemischs B auf der vergleichsweise großen paramagnetischen Suszeptibilität des molekularen Sauerstoffs basiert.

6. Verfahren nach einem der Ansprüche 1 bis 5, **dadurch gekennzeichnet, dass** der stationäre Zielgehalt an molekularem Sauerstoff im Produktgasgemisch B 0,1 bis 6 Vol.-% beträgt.

7. Verfahren nach einem der Ansprüche 1 bis 5, **dadurch gekennzeichnet, dass** der stationäre Zielgehalt an molekularem Sauerstoff im Produktgasgemisch B 0,5 bis 5 Vol.-% beträgt.

8. Verfahren nach einem der Ansprüche 1 bis 5, **dadurch gekennzeichnet, dass** der stationäre Zielgehalt an molekularem Sauerstoff im Produktgasgemisch B 0,1 bis 6 Gew.-% beträgt.

9. Verfahren nach einem der Ansprüche 1 bis 5, **dadurch gekennzeichnet, dass** der stationäre Zielgehalt an molekularem Sauerstoff im Produktgasgemisch B 0,5 bis 5 Gew.-% beträgt.

10. Verfahren nach einem der Ansprüche 1 bis 9, **dadurch gekennzeichnet, dass** die Zufuhr an Frischpropan nur über die Reaktionszone A erfolgt.

11. Verfahren nach einem der Ansprüche 1 bis 10, **dadurch gekennzeichnet, dass** die heterogen katalysierte Dehydrierung in der Reaktionszone A in einem Hordenreaktor durchgeführt wird.

12. Verfahren nach einem der Ansprüche 1 bis 11, **dadurch gekennzeichnet, dass** die heterogen katalysierte Dehydrierung in der Reaktionszone A adiabat betrieben wird.

13. Verfahren nach einem der Ansprüche 1 bis 12, **dadurch gekennzeichnet, dass** die heterogen katalysierte Dehydrierung in der Reaktionszone A autotherm ausgeführt wird.

14. Verfahren nach einem der Ansprüche 1 bis 13, **dadurch gekennzeichnet, dass** die Änderung der Zufuhrrate an Frischpropan unmittelbar in die Reaktionszone A hinein erfolgt.

15. Verfahren nach einem der Ansprüche 1 bis 14, **dadurch gekennzeichnet, dass** die Änderung der Zufuhrrate an Frischpropan unmittelbar in das der Reaktionszone A zugeführte Reaktionsgasgemischeingangsgas hinein erfolgt.

## Claims

1. A process for stably operating a continuous preparation process for obtaining acrolein or acrylic acid or a mixture thereof from propane, in which
A) in a first reaction zone A, propane is subjected in the presence of molecular oxygen to a heterogeneously catalyzed dehydrogenation to obtain a product gas mixture A comprising propane and propylene,
B) if appropriate, product gas mixture A is conducted into a first separation zone A in order to remove a portion or more of constituents other than propane and propylene present in product gas mixture A therefrom and thus to obtain a remaining product gas mixture A' comprising propane and propylene,
C) product gas mixture A or product gas mixture A' is used in a second reaction zone B to charge at least one oxidation reactor and, in the at least one oxidation reactor, propylene present in product gas mixture A or in product gas mixture A' is subjected to a selective heterogeneously catalyzed partial gas phase oxidation with molecular oxygen to give a product gas mixture B comprising acrolein or acrylic acid or a mixture thereof as the target product, unconverted propane, excess molecular oxygen and any unconverted propylene,
D) in a second separation zone B, target product present in product gas mixture B is removed and, from the remaining residual gas comprising propane, molecular oxygen and any unconverted propylene, at least a portion comprising unconverted propane, molecular oxygen and any unconverted propylene is recycled as cycle gas 1 comprising molecular oxygen into reaction zone A in cycle gas mode,
E) fresh propane is fed to the continuous preparation process via at least one of the zones from the group comprising reaction zone A, separation zone A, reaction zone B and separation zone B at a feed rate which has a predefined steady-state value in the stable operating state of the preparation process, and
F) the content of molecular oxygen in product gas mixture B is determined continuously and compared to the steady-state target content of molecular oxygen in product gas mixture B predefined for the stable operating state of the preparation process,
wherein
- in the case that the content of molecular oxygen determined at one time in product gas mixture B is greater than the steady-state target content, after this time, fresh propane is fed to the preparation process at a feed rate which is greater than its steady-state value, and
- in the case that the content of molecular oxygen determined at one time in product gas mixture B is less than the steady-state target content, after this time, fresh propane is fed to the preparation process at a feed rate which is less than its steady-state value.

2. The process according to claim 1, wherein the continuous determination of the content of molecular oxygen in product gas mixture B is based on obtaining a signal which is correlated to the oxygen content of product gas mixture B.

3. The process according to claim 2, wherein the signal is obtained by passing electromagnetic radiation having a wavelength at which molecular oxygen absorbs electromagnetic radiation through product gas mixture B and measuring the unabsorbed fraction of the electromagnetic radiation.

4. The process according to claim 3, wherein the wavelength is from 759.5 nm to 768 nm.

5. The process according to claim 2, wherein the determination of the content of molecular oxygen in product gas mixture B is based on the comparatively great paramagnetic susceptibility of molecular oxygen.

6. The process according to any of claims 1 to 5,
wherein the steady-state target content of molecular oxygen in product gas mixture B is from 0.1 to 6% by volume.

7. The process according to any of claims 1 to 5,
wherein the steady-state target content of molecular oxygen in product gas mixture B is from 0.5 to 5% by volume.

8. The process according to any of claims 1 to 5,
wherein the steady-state target content of molecular oxygen in product gas mixture B is from 0.1 to 6% by weight.

9. The process according to any of claims 1 to 5,
wherein the steady-state target content of molecular oxygen in product gas mixture B is from 0.5 to 5% by weight.

10. The process according to any of claims 1 to 9,
wherein fresh propane is fed only via reaction zone A.

11. The process according to any of claims 1 to 10,
wherein the heterogeneously catalyzed dehydrogenation in reaction zone A is carried out in a tray reactor.

12. The process according to any of claims 1 to 11,
wherein the heterogeneously catalyzed dehydrogenation in reaction zone A is operated adiabatically.

13. The process according to any of claims 1 to 12,
wherein the heterogeneously catalyzed dehydrogenation in reaction zone A is performed autothermally.

14. The process according to any of claims 1 to 13,
wherein the feed rate of fresh propane is changed directly into reaction zone A.

15. The process according to any of claims 1 to 14,
wherein the feed rate of fresh propane is changed directly into the reaction gas mixture input gas fed into reaction zone A.

## Revendications

1. Procédé d'exploitation stable d'un processus de fabrication mis en oeuvre en continu pour la fabrication d'acroléine ou d'acide acrylique ou de leur mélange à partir de propane, selon lequel
A) dans une première zone de réaction A, le propane est soumis à une déshydrogénation sous catalyse hétérogène en présence d'oxygène moléculaire, un mélange gazeux de produits A contenant du propane et du propylène étant obtenu,
B) le mélange gazeux de produits A est éventuellement introduit dans une première zone de séparation afin d'isoler un sous-ensemble ou plus de constituants contenus dans le mélange gazeux de produits A différents du propane et du propylène et ainsi de former un mélange gazeux de produits A' restant qui contient du propane et du propylène,
C) le mélange gazeux de produits A ou le mélange gazeux de produits A' est utilisé dans une seconde zone de réaction B pour l'alimentation d'au moins un réacteur d'oxydation et dans le ou les réacteurs, le propylène contenu dans le mélange gazeux de produits A ou le mélange gazeux de produits A' est soumis à une oxydation sélective partielle en phases gazeuses sous catalyse hétérogène avec de l'oxygène moléculaire pour former un mélange gazeux de produits B contenant de l'acroléine ou de l'acide acrylique ou leur mélange en tant que produit cible, du propane non réagi, de l'oxygène moléculaire en excès et éventuellement du propylène non réagi,
D) dans une seconde zone de séparation B, le produit cible contenu dans le mélange gazeux de produits B est séparé et à partir du gaz résiduel restant contenant du propane, de l'oxygène moléculaire et éventuellement du propylène non réagi, au moins un sous-ensemble contenant du propane non réagi, de l'oxygène moléculaire et éventuellement du propylène non réagi est recyclé dans la zone de réaction A en mode de gaz de recyclage en tant que gaz de recyclage 1 contenant de l'oxygène moléculaire,
E) du propane frais est introduit dans le processus de fabrication mis en oeuvre en continu au niveau d'au moins une des zones du groupe constitué de la zone de réaction A, la zone de séparation A, la zone de réaction B et la zone de séparation B avec un taux d'introduction qui présente une valeur stationnaire fixée à l'état d'exploitation stable du processus de fabrication et
F) la teneur en oxygène moléculaire du mélange gazeux de produits B est calculée en continu et comparée à la teneur cible stationnaire fixée en oxygène moléculaire dans le mélange gazeux de produits B pour l'état d'exploitation stable du processus de fabrication,
**caractérisé en ce que**
- dans le cas où la teneur en oxygène moléculaire dans le mélange gazeux de produits B calculée pour un temps est supérieure à la teneur cible stationnaire, du propane frais est introduit dans le processus de fabrication après ce temps avec un taux d'introduction qui est supérieur à sa valeur stationnaire et
- dans le cas où la teneur en oxygène moléculaire dans le mélange gazeux de produits B calculée pour un temps est inférieure à la teneur cible stationnaire, du propane frais est introduit dans le processus de fabrication après ce temps avec un taux d'introduction qui est inférieur à sa valeur stationnaire.

2. Procédé selon la revendication 1, **caractérisé en ce que** le calcul continu de la teneur en oxygène moléculaire du mélange gazeux de produits B consiste en l'obtention d'un signal qui est corrélé avec la teneur en oxygène du mélange gazeux de produits B.

3. Procédé selon la revendication 2, **caractérisé en ce que** le signal est obtenu par exposition du mélange gazeux de produits B à un rayonnement électromagnétique ayant une longueur d'onde telle que l'oxygène moléculaire absorbe le rayonnement électromagnétique et mesure de la partie non absorbée du rayonnement électromagnétique.

4. Procédé selon la revendication 3, **caractérisé en ce que** la longueur d'onde est de 759,5 nm à 768 nm.

5. Procédé selon la revendication 2, **caractérisé en ce que** le calcul de la teneur en oxygène moléculaire du mélange gazeux de produits B est fondé sur la susceptibilité paramagnétique relativement grande de l'oxygène moléculaire.

6. Procédé selon l'une quelconque des revendications 1 à 5, **caractérisé en ce que** la teneur cible stationnaire en oxygène moléculaire dans le mélange gazeux de produits B est de 0,1 à 6 % en volume.

7. Procédé selon l'une quelconque des revendications 1 à 5, **caractérisé en ce que** la teneur cible stationnaire en oxygène moléculaire dans le mélange gazeux de produits B est de 0,5 à 5 % en volume.

8. Procédé selon l'une quelconque des revendications 1 à 5, **caractérisé en ce que** la teneur cible stationnaire en oxygène moléculaire dans le mélange gazeux de produits B est de 0,1 à 6 % en poids.

9. Procédé selon l'une quelconque des revendications 1 à 5, **caractérisé en ce que** la teneur cible stationnaire en oxygène moléculaire dans le mélange gazeux de produits B est de 0,5 à 5 % en poids.

10. Procédé selon l'une quelconque des revendications 1 à 9, **caractérisé en ce que** l'introduction de propane frais est réalisée uniquement au niveau de la zone de réaction A.

11. Procédé selon l'une quelconque des revendications 1 à 10, **caractérisé en ce que** la déshydrogénation sous catalyse hétérogène dans la zone de réaction A est réalisée dans un réacteur à plateaux.

12. Procédé selon l'une quelconque des revendications 1 à 11, **caractérisé en ce que** la déshydrogénation sous catalyse hétérogène dans la zone de réaction A est réalisée de manière adiabatique.

13. Procédé selon l'une quelconque des revendications 1 à 12, **caractérisé en ce que** la déshydrogénation sous catalyse hétérogène dans la zone de réaction A est réalisée de manière autotherme.

14. Procédé selon l'une quelconque des revendications 1 à 13, **caractérisé en ce que** la modification du taux d'introduction de propane frais a lieu directement dans la zone de réaction A.

15. Procédé selon l'une quelconque des revendications 1 à 14, **caractérisé en ce que** la modification du taux d'introduction de propane frais a lieu directement dans le mélange de gaz réactionnels d'entrée introduit dans la zone de réaction A.
